# EUROPEAN PATENT APPLICATION

(11) **EP 2 447 267 A1**
(43) Date of publication of application: **02.05.2012**
(21) Application number: 10792222.1
(22) Date of filing: 22.06.2010
(51) Int. Cl.: C07D 491/107, C07D 493/10, C08K 5/151, C08L 101/00, C09K 9/02, G02B 5/23

(54) **CHROMENE COMPOUND**

(30) Priority: 25.06.2009 JP 2009150798
(71) Applicant: Tokuyama Corporation, Shunan-shi, Yamaguchi 745-0053 (JP)
(72) Inventor: TAKAHASHI Toshiaki, Shunan-shi YAMAGUCHI 745-0053 (JP); TAKENAKA Junji, Shunan-shi YAMAGUCHI 745-0053 (JP); TERANISHI Kazuhiro, Shunan-shi YAMAGUCHI 745-0053 (JP)
(74) Representative: Adam, Holger
(86) International application number: PCT/JP2010/060936
(87) International publication number: WO 2010/150905

(57) **Abstract**

A chromene compound which contains a skeleton represented by the following formula (1) and has high color optical density at the time of exposure, double peak characteristic, little initial coloration, a practical fading speed and durability. 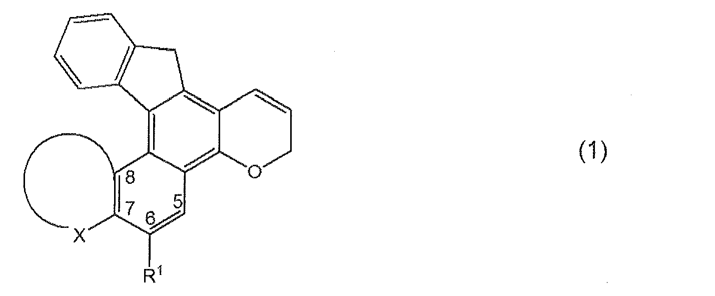 (wherein R¹ is an electron donor group having a Hammett constant of less than 0, the ring including X is a hetero ring formed together with the 7-position and 8-position carbon atoms, and X is an oxygen atom, sulfur atom or group represented by =NR² directly bonded to the 7-position carbon atom.)

## Description

### TECHNICAL FIELD

The present invention relates to a novel chromene compound, a precursor thereof and use of the chromene compound.

### BACKGROUND ART

Photochromism is the reversible function of a certain compound that it changes its color swiftly upon exposure to light including ultraviolet light such as sunlight or light from a mercury lamp and returns to its original color when it is put in the dark by stopping its exposure to light. A compound having this property is called "photochromic compound" and used as a material for photochromic plastic lenses which are photochromic optical articles.

The following properties are required for the photochromic compound used for the above purpose:
(i) the degree of coloration at a visible light range before ultraviolet light is applied (to be referred to as "initial coloration" hereinafter)) should be low;
(ii) the degree of coloration upon exposure to ultraviolet light (to be referred to as "color optical density" hereinafter) should be high;
(iii) the speed from the time when the application of ultraviolet light is started to the time when the color optical density reaches saturation (to be referred to as "color development sensitivity" hereinafter) should be high;
(iv) the speed from the stoppage of the application of ultraviolet light to the time when the compound returns to its original state (to be referred to as "fading speed" hereinafter) should be high;
(v) the repeat durability of this reversible function should be high; and
(vi) the solubility in a monomer compound which will become a host material after curing of the photochromic compound should be high so that its dispersibility in the host material in use becomes high.

It has been desired that the photochromic plastic lenses develop a color of a neutral tint such as grey or brown. What color is developed depends on the photochromic compound as a matter of course, which is a very important factor. When the color is to be adjusted by mixing together a plurality of photochromic compounds, there occur various problems such as a color change at the time of color development and fading (to be referred to as "color shift" hereinafter) due to the different characteristic properties of the photochromic compounds that are mixed together and a color change at the time of deterioration due to the difference in durability. To solve the above problems, a photochromic compound which by itself has two or more absorption wavelengths at a visible range when developing a color to develop a color of a neutral tint (to be referred to as "double peak compound" hereinafter) is important.

As the double peak compound, there are known a chromene compound represented by the following formula (A) (refer to a pamphlet of International Laid-Open WO01/19813), a chromene compound represented by the following formula (B) or (C) (refer to a pamphlet of International Laid-Open WO03/025638), a chromene compound represented by the following formula (D) (refer to a pamphlet of International Laid-Open WO03/044022), a chromene compound represented by the following formula (E) (refer to the specification of US Patent No. 5698141) and a chromene compound represented by the following formula (F) (refer to a pamphlet of International Laid-Open WO05/028465) These chromene compounds have two absorption wavelengths at a visible range when developing a color and exhibit excellent characteristic properties.

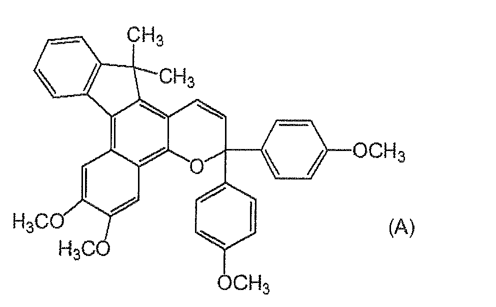

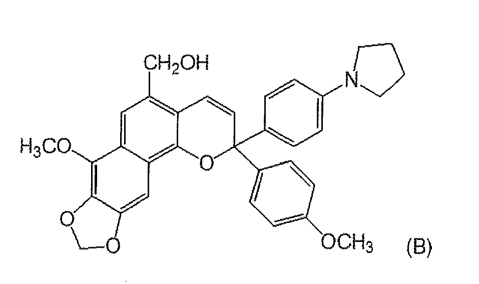

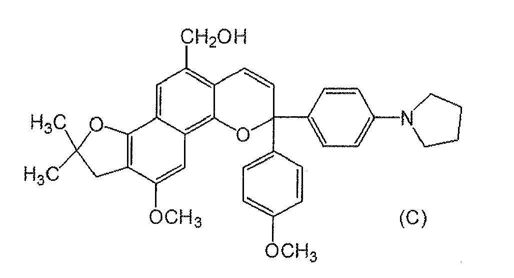

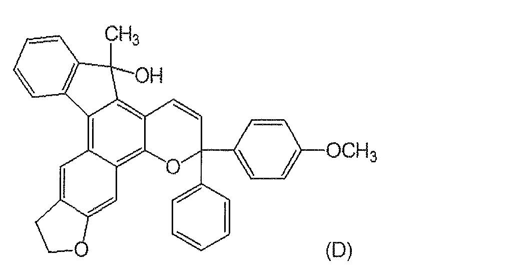

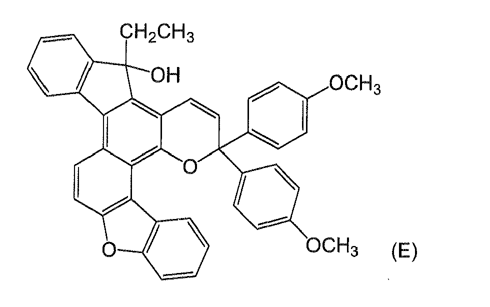

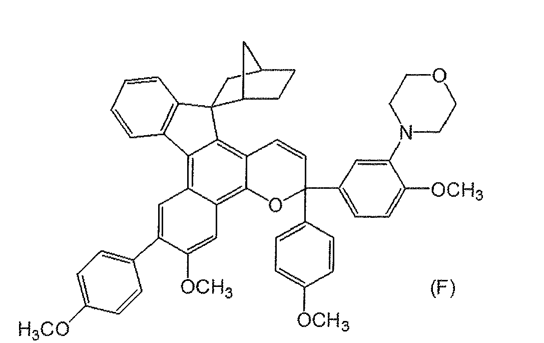

### DISCLOSURE OF THE INVENTION

In the field of photochromic plastic lenses, the requirements for photochromic properties, especially high fading speed when a photochromic plastic lens moves from outdoors to indoors and transparency when a user wears a photochromic plastic lens indoors (little initial coloration) are becoming stronger and stronger each year. Therefore, the development of a photochromic compound which satisfies all the above requirements (i) to (vi) at a higher level than the chromene compounds of the prior art is desired. When the color is to be adjusted by mixing together a plurality of photochromic compounds, for example, chromene compounds, it is known that a photochromic compound which develops a yellow color is generally inferior in durability to a photochromic compound which develops another color, for example, a blue color. Therefore, a compound which has a higher color optical density at a yellow range (430 to 530 nm) than a color optical density at a blue range (550 to 650 nm) is desired as the double peak compound (in the double peak compound, the ratio of the yellow color optical density to the blue color optical density may be referred to as "double peak characteristic" hereinafter). In consideration of these characteristic properties, the chromene compounds of the prior art have room for the improvement of the following points.

For example, although the chromene compound represented by the above formula (A) has practical levels of color optical density and double peak characteristic, it has room for improvement as it has a low fading speed. The chromene compound represented by the above formula (B), (C), (D) or (E) also has room for improvement as it does not have satisfactory double peak characteristic. Further, although the chromene compound represented by the above formula (F) is excellent in double peak characteristic and has practical levels of color optical density and fading speed as it is a compound whose 7-position carbon atom is substituted by a specific aryl group, it has room for improvement as the end portion of its absorption spectrum (to be referred to as "absorption end" hereinafter) goes beyond 420 nm into the visible range with the result of large initial coloration.

Therefore, it is an object of the present invention to provide a novel photochromic compound (chromene compound) which has little initial coloration, high color optical density when it is exposed to light, high color development sensitivity, high fading speed and high durability and exhibits excellent double peak characteristic.

It is another obj ect of the present invention to provide a naphthol compound which is an intermediate for the production of the chromene compound of the present invention.

Other objects and advantages of the present invention will become apparent from the following description.

The inventors of the present invention conducted intensive studies to attain the above objects and found that the double peak characteristic can be enhanced, the wavelength of the absorption end can be made short and the initial coloration can be reduced by increasing the electron donating ability of the 7-position substituent in the indenonaphthopyran skeleton and also that when the electron donating ability of the 7-position substituent is increased, the fading speed and the durability become lower in proportion to this.

Then, the inventors thought if the above disadvantage could be improved by adjusting the electron donating ability of the 7-position substituent while retaining the above advantage and investigated the introduction of various substituents. They further investigated the substituents at the 6-position and the 8-position adjacent to the 7-position.

As a result, they found that the above objects can be attained by a chromene compound in which a hetero atom is directly bonded to the 7-position carbon atom, a hetero ring including the 7-position and 8-position carbon atoms and the hetero atom is formed in the indenonaphthopyran skeleton, and further an electron donor group having a Hammett constant σₚ of less than 0 is introduced into the adjacent 6-position. The present invention has been accomplished based on this finding.

That is, firstly, the present invention is a chromene compound having a skeleton represented by the following formula (1): 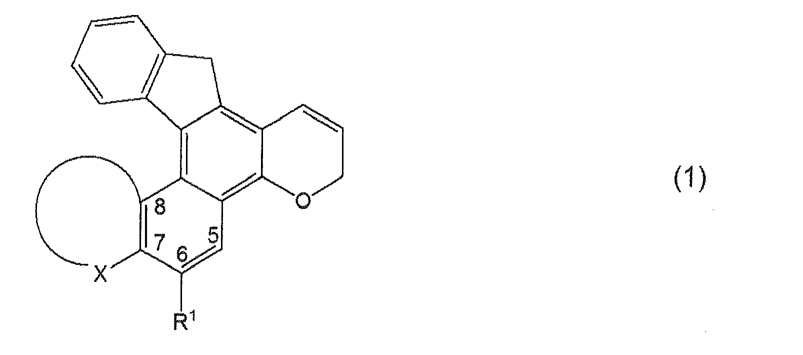 wherein R¹ is an electron donor group having a Hammett constant σₚ of less than 0, and the ring portion represented by the following formula (2) is a hetero ring: 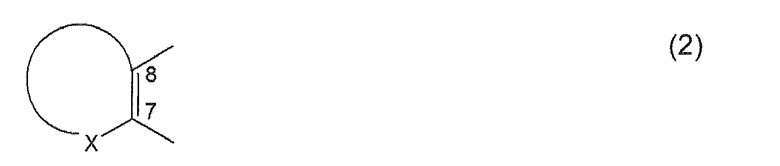 wherein X is an oxygen atom, sulfur atom or group represented by the following formula (3) which is directly bonded to the 7-position carbon atom: 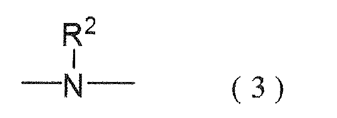 wherein R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom.

Secondly, the present invention is a photochromic curable composition which contains a chromene compound having a skeleton represented by the above formula (1) and a polymerizable monomer.

Thirdly, the present invention is a photochromic optical article which has a polymer molded product containing a chromene compound having a skeleton represented by the above formula (1) dispersed therein as a constituent member.

In the fourth place, the present invention is an optical article having an optical substrate whose surface is at least partially coated with a polymer film as a constituent part, wherein the polymer film contains the chromene compound having a skeleton represented by the above formula (1) dispersed therein.

Finally, the present invention is a naphthol compound represented by the following formula (8): 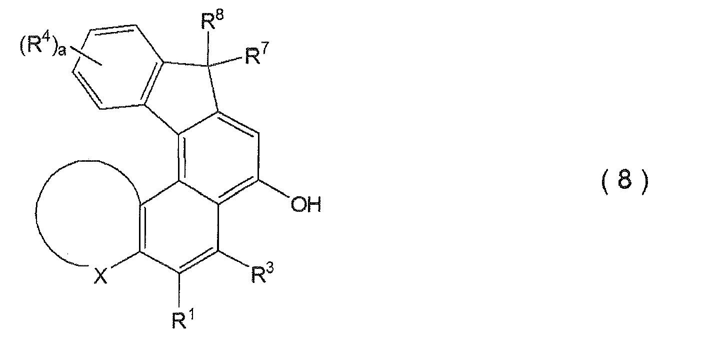 wherein a, R¹, R³, R⁴, R⁷, R⁸ and a ring portion including X are as defined in the formula (6) in claim 3.

### BEST MODE FOR CARRYING OUT THE INVENTION

The chromene compound of the present invention is a chromene compound having a skeleton represented by the above formula (1). The following substituents will be described hereinbelow.

### (group R¹)

The group R¹ in the above formula (1) is an electron donor group having a Hammett constant σₚ of less than 0.

The Hammett constant σₚ is defined based on the Hammett equation that quantifies the electric effect of a substituent bonded to an π electron system on the basis of the dissociation constant Ka of p-substituted benzoic acid. A substituent having a σₚ of 0 is a hydrogen atom, and a substituent having a σₚ of less than 0, that is, a negative number is a substituent having higher electron donating ability than the hydrogen atom.

Examples of the electron donor group R¹ having a σₚ of less than 0 include hydroxyl group (σₚ = -0.37), alkyl group, cycloalkyl group and alkoxy group.

The group R¹ may be an aralkyl group, aryloxy group, aryl group, amino group or heterocyclic group having a nitrogen atom as a member hetero atom and bonded to the 6-position carbon atom via the nitrogen atom. These groups may be substituted by an electron donor substituent having a σₚ of less than 0. The aralkyl group, aryloxy group, aryl group, amino group or heterocyclic group having a nitrogen atom as a member hetero atom and bonded to the 6-position carbon atom via the nitrogen atom is an electron donor group having a σₚ of less than 0 when it has no substituent. Therefore, when these groups are substituted by an electron donating group having a σₚ of less than 0, they become groups having a σₚ of less than 0.

A detailed description is subsequently given of the above electron donor group having a Hammett constant σₚ of less than 0.

The above alkyl group is generally a group having a σₚ of -0.20 to -0.10 and particularly preferably an alkyl group having 1 to 8 carbon atoms in the present invention. Preferred examples of the alkyl group include methyl group (σₚ =-0.14), ethyl group (σₚ =-0.13), n-propyl group (σₚ =-0.12), isopropyl group, n-butyl group, sec-butyl group, tert-butyl group (σₚ =-0.15), pentyl group, hexyl group, heptyl group and octyl group.

The cycloalkyl group is generally a group having a σₚ of less than 0 and particularly preferably a cycloalkyl group having 3 to 8 carbon atoms in the present invention. Preferred examples of the cycloalkyl group include cyclopropyl group, cyclobutyl group, cyclopentyl group, cyclohexyl group (σₚ =-0.16), cycloheptyl group and cyclooctyl group.

The alkoxy group is generally a group having a σₚ of -0.3 to -0.2 and particularly preferably an alkoxy group having 1 to 8 carbon atoms in the present invention. Preferred examples of the alkoxy group include methoxy group (σₚ =-0.28), ethoxy group (σₚ =-0.21), n-propoxy group (σₚ =-0.26), isopropoxy group, n-butoxy group, sec-butoxy group and tert-butoxy group.

The aralkyl group is generally a group having a σₚ of less than 0 and particularly preferably an aralkyl group having 7 to 11 carbon atoms in the present invention. Preferred examples of the aralkyl group include benzyl group, phenylethyl group, phenylpropyl group, phenylbutyl group and naphthylmethyl group. One or more hydrogen atoms on the benzene ring of the aralkyl group may be substituted by a group having a σₚ of less than 0, for example, the above alkyl group, cycloalkyl group, alkoxy group, aralkyl group or aryloxy group. Since the aralkyl group having no substituent has a σₚ of less than 0, if the aralkyl group is substituted by a group having a σₚ of less than 0, it has a σₚ of less than 0.

The aryloxy group is generally a group having a σₚ of -0.6 to -0.4 and particularly preferably an aryloxy group having 7 to 11 carbon atoms in the present invention. Preferred examples of the aryloxy group include phenyloxy group (σₚ =-0.49) and naphthyloxy group. One or more hydrogen atoms on the benzene ring of the aryloxy group may be substituted by a group having a σₚ of less than 0, for example, the above alkyl group, cycloalkyl group, alkoxy group, aralkyl group or aryloxy group. The aryloxy group substituted by any one of these groups has a σₚ of less than 0 as well.

The aryl group is generally a group having a σₚ of -0.1 to -0.01 and particularly preferably an aryl group having 6 to 14 carbon atoms in the present invention. Preferred examples of the aryloxy group include phenyl group (σₚ =-0_{.}01) and 1-naphthol group (σₚ =-0.08). One or more hydrogen atoms on the benzene ring of the aryl group may be substituted by a group having a σₚ of less than 0, for example, the above alkyl group, cycloalkyl group, alkoxy group, aralkyl group or aryloxy group. The aryl group substituted by any one of these groups has a σₚ of less than 0 as well.

The amino group is generally a group having a σₚ of -1.00 to -0.10. Preferred examples of the amino group include primary amino group (σₚ =-0.66), secondary amino group and tertiary amino group. The substituent of the secondary or tertiary amino group is preferably a group having a σₚ of less than 0, as typified by alkyl group and aryl group. The amino group substituted by any one of them has a σₚ of less than 0 as well. Preferred examples of the secondary amino group and the tertiary amino group include alkylamino groups such as methylamino group (σₚ =-0.77) and ethylamino group; dialkylamino groups such as dimethylamino group (σₚ =-0.83) and diethylamino group; arylamino groups such as phenylamino group (σₚ =-0.11); and diarylamino groups such as diphenylamino group.

The heterocyclic group having a nitrogen atom as member hetero atom and bonded to the 6-position carbon atom via the nitrogen atom is generally a group having a σₚ of -1.00 to -0.40. Preferred examples of the heterocyclic group include morpholino group (σₚ =-0.50), piperidino group (σₚ =-0.83), pyrrolidinyl group, piperazino group, N-methylpiperazino group and indolinyl group. Further, these heterocyclic groups may have a group having a σₚ of less than 0 as a substituent. Examples of the substituent include alkyl groups such as methyl group. Even when the heterocyclic group has such a substituent, it has a σₚ of less than 0. Examples of the heterocyclic group having a substituent include 2,6-dimethylmorpholino group, 2,6-dimethylpiperidino group and 2,2,6,6-tetramethylpiperidino group.

In the present invention, since the double peak characteristic of the chromene compound becomes especially high, the group R¹ is preferably an electron donor group having a σₚ of -1.00 to -0.20 out of the groups enumerated above. More specifically, the group R¹ is preferably a hydroxyl group, or alkoxy group, aryloxy group, amino group or heterocyclic group having a nitrogen atom as a member hetero atom and bonded to the 6-position carbon atom via the nitrogen atom, the latter four having a σₚ of -1.00 to -0.20. Out of these, an alkoxy group and a heterocyclic group having a nitrogen atom as a member hetero atom and bonded to the 6-position carbon atom via the nitrogen atom, all of which have a σₚ of -0.60 to -0.20, are particularly preferred because they improve the fading speed and initial coloration due to the absorption end. More specifically, the particularly preferred substituent is selected from methoxy group and morpholino group.

### (hetero ring)

The ring portion represented by the following formula (2) is a hetero ring including the 7-position and 8-position carbon atoms and X. The number of member atoms forming the hetero ring is preferably 5 to 7. That is, the number of member atoms indicates the number of atoms forming the ring and does not include the number of atoms of a substituent substituting the formed hetero ring. Since the chromene compound of the present invention forms the above hetero ring, it becomes a chromene compound having excellent photochromic properties.

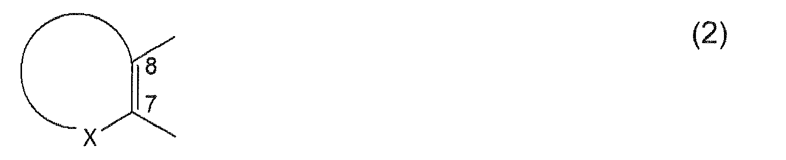

In the above formula (2), X is an oxygen atom, sulfur atom or group represented by the following formula (3) bonded to the 7-position carbon atom.

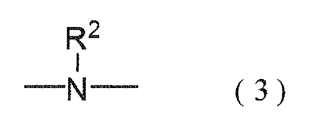

In the above formula (3), the group R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom.

Preferred examples of the alkyl group, cycloalkyl group, alkoxy group, aralkyl group, aryloxy group, aryl group, amino group and heterocyclic group having a nitrogen atom as a member hetero atom and bonded to the nitrogen atom in the above formula (3) via the above nitrogen atom are the same as those enumerated for the above group R¹.

The haloalkyl group is preferably a haloalkyl group having 1 to 8 carbon atoms as exemplified by trifluoromethyl group and 2,2,2-trifluoroethyl group.

The alkenyl group is preferably an alkenyl group having 2 to 9 carbon atoms as exemplified by allyl group, propenyl group, 1-butenyl group and 2-butenyl group.

The alkynyl group is preferably an alkynyl group having 2 to 9 carbon atoms as exemplified by propargyl group and 1-pentinyl group.

The alkylcarbonyl group is preferably an alkylcarbonyl group having 2 to 9 carbon atoms as exemplified by methylcarbonyl group and ethylcarbonyl group.

The alkoxycarbonyl group is preferably an alkoxycarbonyl group having 2 to 9 carbon atoms as exemplified by methoxycarbonyl group and ethoxycarbonyl group.

Examples of the halogen atom are fluorine atom, chlorine atom, bromine atom and iodine atom.

To obtain higher double peak characteristic and fading speed, it is important that, out of the above groups, X should have electron donating ability, and X is preferably an oxygen atom or a group represented by the above formula (3). In the group represented by the above formula (3), the group R² is preferably a group having a Hammett constant σₚ of -0.20 to 1.00 as exemplified by hydrogen atom, alkyl group, haloalkyl group, cyano group, nitro group and halogen atom from the viewpoints of absorption end and fading speed. From the viewpoint of the double peak characteristic, hydrogen atom, alkyl group, haloalkyl group and halogen atom are more preferred. More specifically, hydrogen atom, methyl group, trifluoromethyl group and fluorine atom are more preferred.

Further, out of hetero rings represented by the above formula (2), a hetero ring represented by the following formula (4) is particularly preferred so as to obtain a chromene compound having excellent photochromic properties. This preferred hetero ring will be described hereinbelow.

### (preferred hetero ring)

In the present invention, the hetero ring represented by the above formula (2) is preferably a 5 to 7 membered hetero ring represented by the following formula (4).

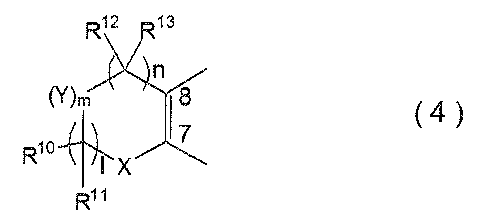

In this case, the skeleton represented by the above formula (1) is represented by the following formula (5).

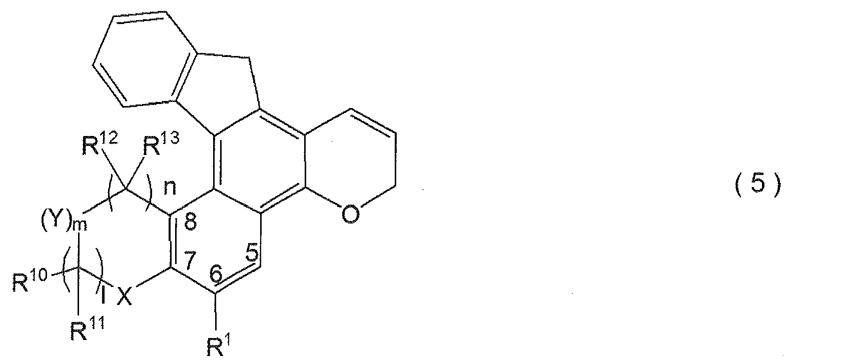

In the above formula, R¹ is as defined in the above formula (1). Therefore, preferred R¹ is the same as the group explained for the above group R¹.

In the above formula, X is as defined in the above formula (2). Therefore, preferred X is the same as the group explained for the above hetero ring.

Y is an oxygen atom, sulfur atom or group represented by the following formula (3).

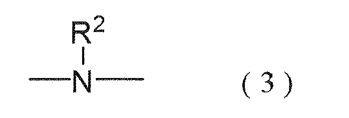

R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom. This R² is the same as the group explained for the above hetero ring. That is, Y is the same as X in the hetero ring represented by the above formula (2).

Preferred examples of Y are the same as those enumerated for X. "m" indicating the number of Y's is preferably 0 or 1 from the viewpoints of double peak characteristic and fading speed, particularly preferably 0 from the viewpoint of double peak characteristic.

R¹⁰, R¹¹, R¹² and R¹³ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a carbon atom bonded thereto via the nitrogen atom, cyano group, nitro group, formyl group, hydroxycarbonyl group, alkylcarbonyl group, alkoxycarbonyl group or halogen atom.

Preferred examples of the alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a carbon atom bonded thereto via the nitrogen atom, alkylcarbonyl group, alkoxycarbonyl group and halogen atom are the same as those enumerated for the group R².

R¹⁰ and R¹¹, or R¹² and R¹³ may be bonded together to form a carbonyl group or aliphatic hydrocarbon ring together with the carbon atom bonded thereto.

The aliphatic hydrocarbon ring formed by bonding together R¹⁰ and R¹¹, or R¹² and R¹³ is preferably an aliphatic hydrocarbon ring having 3 to 10 carbon atoms as exemplified by cyclopropane ring, cyclobutane ring, cyclopentane ring, cyclohexane ring, cycloheptane ring and cyclooctane ring. One or more hydrogen atoms on the aliphatic hydrocarbon ring may be substituted by the above alkyl group, haloalkyl group, alkoxy group or halogen atom.

"1" is an integer of 0 to 4, "m" is an integer of 0 to 1, "n" is an integer of 0 to 4, and (1+m+n) is an integer of 2 to 4.

When "m" is 0, "1" is an integer of 0 to 4, and "n" is an integer of 0 to 4 ((1+n) is an integer of 2 to4). In this case, adjacent R¹⁰ and R¹⁰ (1 ≠ 0 and 1), R¹⁰ and R¹² (1 ≠ 0 and n ≠ 0) or R¹² and R¹² (n ≠ 0 and 1) may be bonded together to form an aliphatic hydrocarbon ring or an aromatic hydrocarbon ring together with a carbon atom bonded thereto.

When "m" is 1, "1" is an integer of 0 to 3, "n" is an integer of 0 to 3, and (1+n) is an integer of 2 to 3. In this case, adjacent R¹⁰ and R¹⁰ (1 ≠ 0 and 1) or adjacent R¹² and R¹² (n ≠ 0 and 1) may be bonded together to form an aliphatic hydrocarbon ring or an aromatic hydrocarbon ring together with a carbon atom bonded thereto.

The aliphatic hydrocarbon ring formed by bonding together adjacent R¹⁰ and R¹⁰, R¹⁰ and R¹², or R¹² and R¹² is preferably an aliphatic hydrocarbon ring having 3 to 10 carbon atoms as exemplified by cyclopropane ring, cyclobutane ring, cyclopentane ring, cyclohexane ring, cycloheptane ring and cyclooctane ring. One or more hydrogen atoms on the aliphatic hydrocarbon ring may be substituted by the above alkyl group, haloalkyl group, alkoxy group or halogen atom.

The aromatic hydrocarbon ring formed by bonding together adjacent R¹⁰ and R¹⁰, R¹⁰ and R¹², or R¹² and R¹² is preferably an aromatic hydrocarbon ring having 6 to 14 carbon atoms as exemplified by benzene ring and naphthalene ring. One or more hydrogen atoms on the aliphatic hydrocarbon ring may be substituted by the above alkyl group, haloalkyl group, alkoxy group or halogen atom.

Preferred examples of R¹⁰ to R¹³ include hydrogen atom, alkyl group, haloalkyl group, alkoxy group and halogen atom, and R¹⁰ and R¹¹, or R¹² and R¹³ are bonded together to form a carbonyl group together with a carbon atom bonded thereto. Out of these substituents, R¹⁰ and R¹¹ are each preferably a hydrogen atom, alkyl group or alkoxy group from the viewpoint of double peak characteristic, as exemplified by hydrogen atom, methyl group and methoxy group. Out of these substituents, R¹² and R¹³ are each preferably a hydrogen atom, haloalkyl group or halogen atom from the viewpoint of double peak characteristic, and R¹⁰ and R¹¹, or R¹² and R¹³ are preferably bonded together to form a carbonyl group together with a carbon atom bonded thereto, as exemplified by hydrogen atom, trifluoromethyl group, fluorine atom and carbonyl group.

As for "1" to "n", (1+m+n) is preferably 2 to 3 from the viewpoints of fading speed and double peak characteristic. (1+m+n) is particularly preferably 2 from the viewpoint of fading speed and 3 from the viewpoint of double peak characteristic.

Particularly preferred examples of the hetero ring represented by the above formula (2) are given below. In the following formulas, carbon atoms denoted by 7 and 8 are 7-position and 8-position carbon atoms in the above formula (1), respectively.

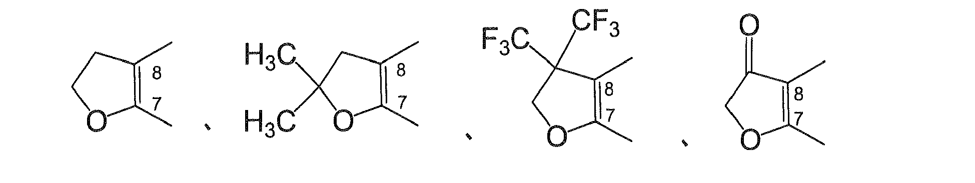

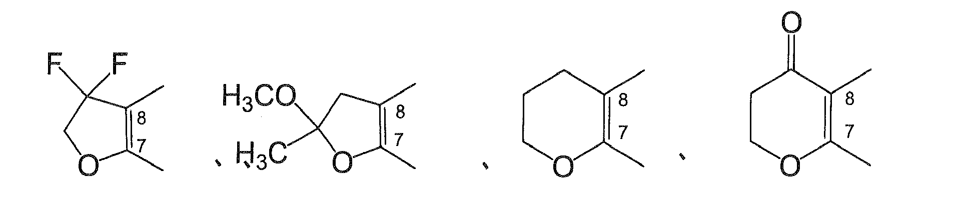

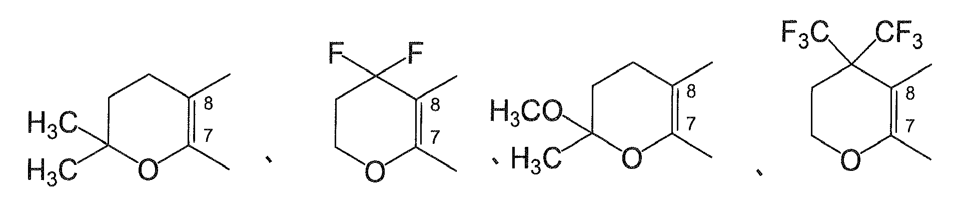

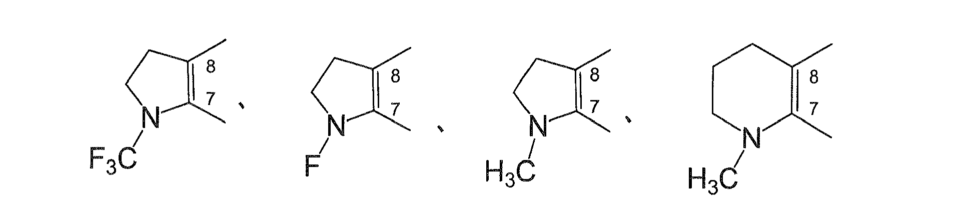

Since the chromene compound of the present invention has an indenonaphthopyran skeleton as represented by the above formula (1) or (5) and the above groups as the 6-position, 7-position and 8-position substitutes, it exhibit excellent photochromic properties. Therefore, although substituents at other positions are not particularly limited, the following chromene compound having other substituents is particularly preferred as the chromene compound having excellent photochromic properties. A description is subsequently given of this preferred chromene compound.

### (preferred chromene compound)

Out of chromene compounds having a skeleton represented by the above formula (1), a chromene compound represented by the following formula (6) is preferred.

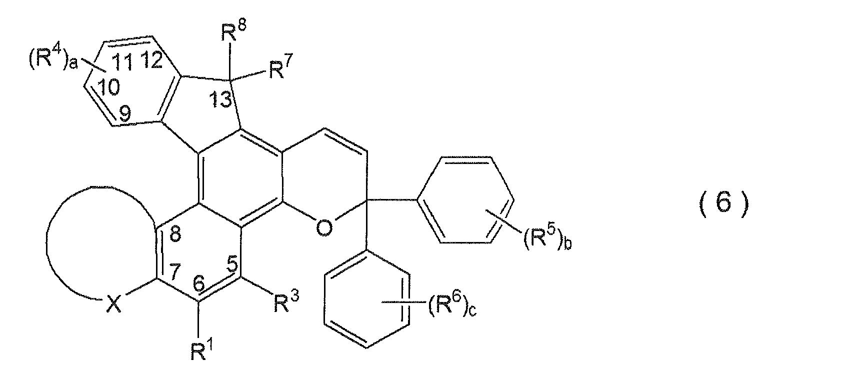

In the above formula (6), R¹, the ring portion represented by the following formula and X are as defined in the above formula (1). Other groups will be described hereinbelow.

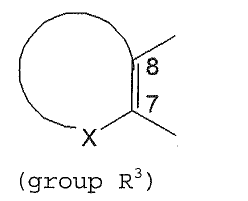

In the chromene compound of the above formula (6), the 5-position substituent R³ is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 5-positon carbon atom, cyano group, nitro group, formyl group, hydroxycarbonyl group, alkylcarbonyl group, alkoxycarbonyl group or halogen atom.

Preferred examples of the above alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 5-positon carbon atom, alkylcarbonyl group, alkoxycarbonyl group and halogen atom are the same as those enumerated for the group R²

In the present invention, the group R³ is involved in fading speed. To accelerate the fading speed, R³ preferably has a stereoscopically small substituent. To this end, the group R³ is particularly preferably a hydrogen atom.

### (group R⁴)

In the chromene compound represented by the above formula (6), the 9- to 12-position substituent R⁴ is a hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a benzene ring bonded thereto via the nitrogen atom, cyano group, nitro group, formyl group, hydroxycarbonyl group, alkylcarbonyl group, alkoxycarbonyl group or halogen atom.

Preferred examples of the above alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a benzene ring bonded thereto via the nitrogen atom, alkylcarbonyl group, alkoxycarbonyl group and halogen atom are the same as those enumerated for the group R².

"a" denotes the number of R⁴' s and an integer of 0 to 4. When "a" is 2 or more, R⁴' s may be the same or different.

In the present invention, the group R⁴ is involved in fading speed. When "a" is 0 (hydrogen atom), the group R⁴ is preferably an electron absorbing group. When the group R⁴ is an electron absorbing group, it is preferably bonded to the 11-position carbon atom so as to accelerate the fading speed. The particularly preferred electron absorbing group is a cyano group or haloalkyl group, more specifically, cyano group or trifluoromethyl group.

### (groups R⁵ and R⁶)

In the chromene compound of the above formula (6), R⁵ and R³ are each independently a hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a benzene ring bonded thereto via the nitrogen atom, cyano group, nitro group, formyl group, hydroxycarbonyl group, alkylcarbonyl group, alkoxycarbonyl group or halogen atom.

Preferred examples of the above alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to a benzene ring bonded thereto via the nitrogen atom, alkylcarbonyl group, alkoxycarbonyl group and halogen atom are the same as those enumerated for the group R².

"b" and "c" denote the numbers of the substituents R⁵' s and R⁶' s, respectively, and are each independently an integer of 0 to 5. When "b" and "c" are each 2 or more, R⁵' s and R⁶' s may be the same or different.

In the present invention, the substituents R⁵ and R⁶ are involved in double peak characteristic and fading speed. Although the numbers and positions of the substituents are not particularly limited, they are preferably existent at the p-position of a benzene ring bonded thereto in order to enhance the double peak characteristic and obtain a high fading speed. When "b" or "c" is 0 (hydrogen atom), the preferred substituents are each an alkyl group, alkoxy group, amino group or heterocyclic group having a nitrogen atom as a member hetero atom and bonded to a benzene bonded thereto via the nitrogen atom, as exemplified by hydrogen atom, methyl group, methoxy group, dimethylamino group and morpholino group. From the viewpoint of the double peak characteristic, the substituents are each preferably a hydrogen atom, alkyl group, alkoxy group or heterocyclic group having a nitrogen atom as a member hetero atom and bonded to a benzene ring bonded thereto via the nitrogen atom, as exemplified by hydrogen atom, methyl group, methoxy group and morpholino group.

### (groups R⁷ and R⁸)

In the chromene compound represented by the above formula (6), the 13-position groups R⁷ and R⁸ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to the 13-position carbon atom via the nitrogen atom, cyano group, nitro group, formyl group, hydroxycarbonyl group, alkylcarbonyl group, alkoxycarbonyl group or halogen atom.

Preferred examples of the above alkyl group, haloalkyl group, alkenyl group, alkynyl group, cycloalkyl group, alkoxy group, aralkyl group, aryloxy group, aryl group, amino group, heterocyclic group having a nitrogen atom as a member hetero atom and bonded to the 13-position carbon atom via the nitrogen atom, alkylcarbonyl group, alkoxycarbonyl group and halogen atom are the same as those enumerated for the group R²

In the chromene compound represented by the above formula (6), R⁷ and R⁸ may be bonded together to form a carbonyl group or aliphatic hydrocarbon ring together with the 13-position carbon atom.

The number of member carbon atoms of the aliphatic hydrocarbon ring is preferably 4 to 20, more preferably 4 to 15 from the viewpoints of color optical density and fading speed. From the viewpoint of fading speed, the number of member carbon atoms is particular preferably 4 to 12. This aliphatic hydrocarbon ring may have at least one substituent selected from alkyl group, haloalkyl group, cycloalkyl group, alkoxy group, amino group, aralkyl group, aryl group and halogen atom. Preferred examples of the alkyl group, haloalkyl group, cycloalkyl group, alkoxy group, amino group, aralkyl group, aryl group and halogen atom as substituents are the same as those enumerated for the group R². Out of these, an alkyl group is preferred from the viewpoints of color optical density and fading speed, as exemplified by methyl group.

In the present invention, preferred substituents R⁷ and R⁸ are each selected from alkyl group, alkoxy group and hydroxyl group, and R⁷ and R⁸ are bonded together to form an aliphatic hydrocarbon ring together with the 13-position carbon atom. An example of the alkyl group is a methyl group, and an example of the alkoxy group is a methoxy group. Out of the above preferred substituents, R⁷ and R⁸ are preferably bonded together to form an aliphatic hydrocarbon ring together with the 13-position carbon atom so as to reduce color development by heat at room temperature under no exposure to light (this color development will be referred to as "initial coloration due to thermochromism" hereinafter) and accelerate the fading speed while retaining the double peak characteristic. The aliphatic hydrocarbon ring is preferably a single ring having 4 to 20 member carbon atoms, bicyclo ring or tricyclo ring. Specific examples of the aliphatic hydrocarbon ring include single rings such as cyclobutane ring, cyclopentane ring, cyclohexane ring, cycloheptane ring, cyclooctane ring, cyclononane ring, cyclodecane ring and 3,3,5,5-tetramethylcyclohexane ring, bicyclo rings such as bicyclo[2,2,1]heptane ring, bicyclo[3,2,1]octane ring and bioyclo[3,3,1]nonane ring, and tricyclo rings such as adamantane ring.

Out of these, a single ring formed by bonding together the groups R⁷ and R⁸ exhibits a particularly excellent effect. Specific examples of the single ring include cyclobutane ring, cyclopentane ring, cyclohexane ring, cycloheptane ring, cyclooctane ring, cyclononane ring, cyclodecane ring and 3,3,5,5-tetramethylcyclohexane ring.

Out of the above single rings, cyclooctane ring and 3,3,5,5-tetramethylcyclohexane ring are particularly preferred.

In the chromene compound of the above formula (6), the hetero ring including 7-position, 8-position and X is preferably a 5- to 7-membered hetero ring represented by the above formula (4). In this case, the chromene compound is represented by the following formula (7).

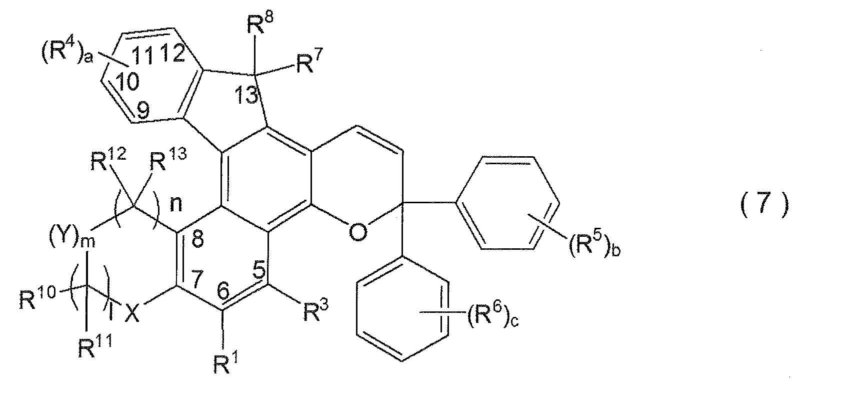

In the above formula, R¹, R³, R⁴, R⁵, R⁶, R⁷, R⁸, "a", "b" and "c" are as defined in the above formula (6), and the ring portion represented by the following formula and X, Y, R¹⁰, R¹¹, R¹², R¹³, "l", "m" and "n" are as defined in the above formula (5).

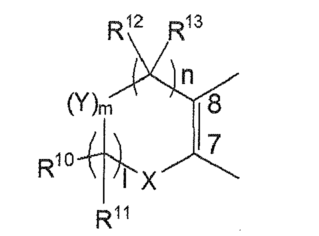

Since the groups in the above formula (7) are as defined in the above formulas (5) and (6), preferred groups, the number of the substituents and the positions of the substituents are the same as those explained for the basic skeleton represented by the above formula (5) and the chromene compound represented by the above formula (6) as a matter of course.

### (preferred examples of chromene compound)

In the present invention, preferred examples of the chromene compound are the following compounds.

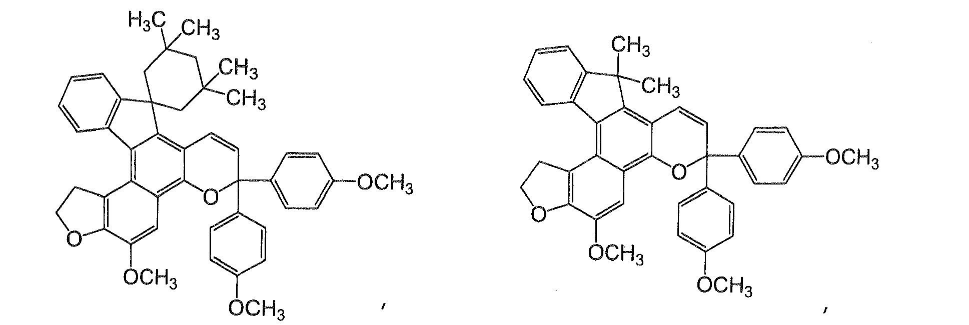

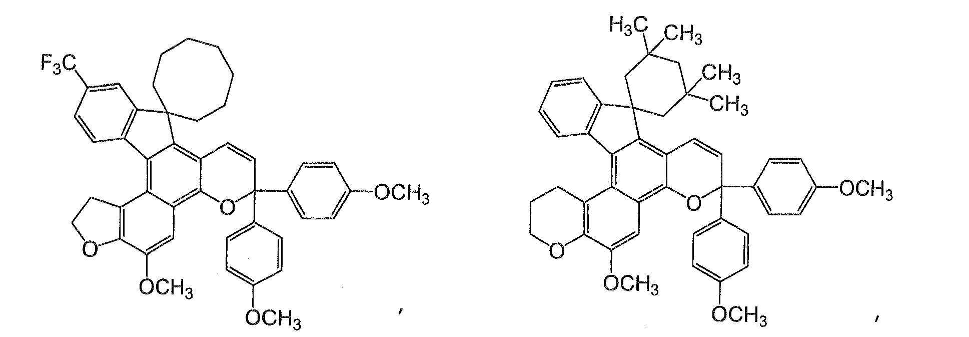

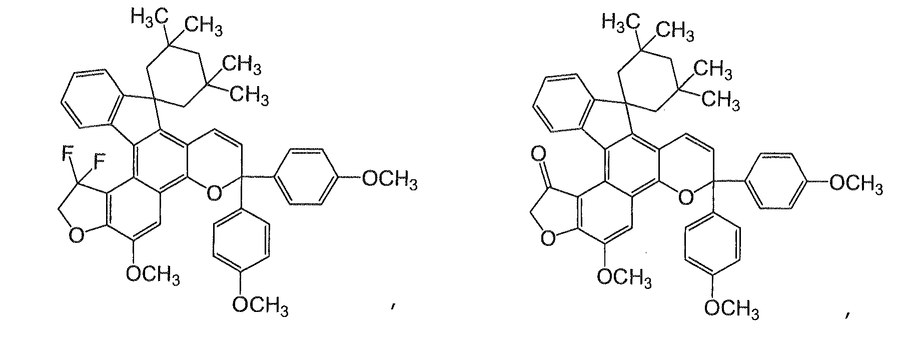

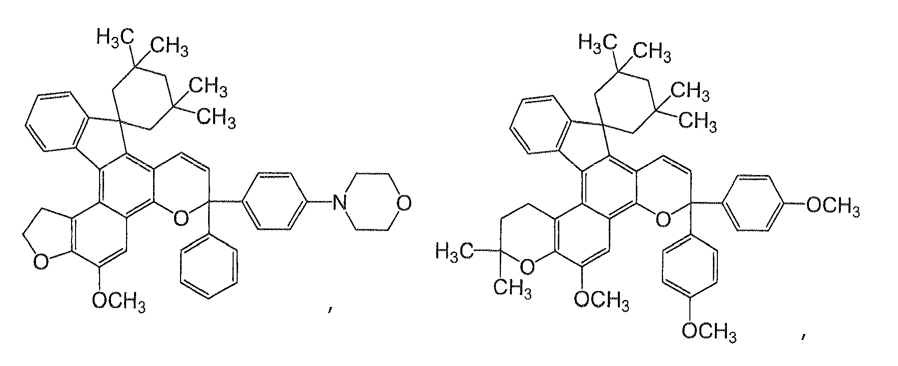

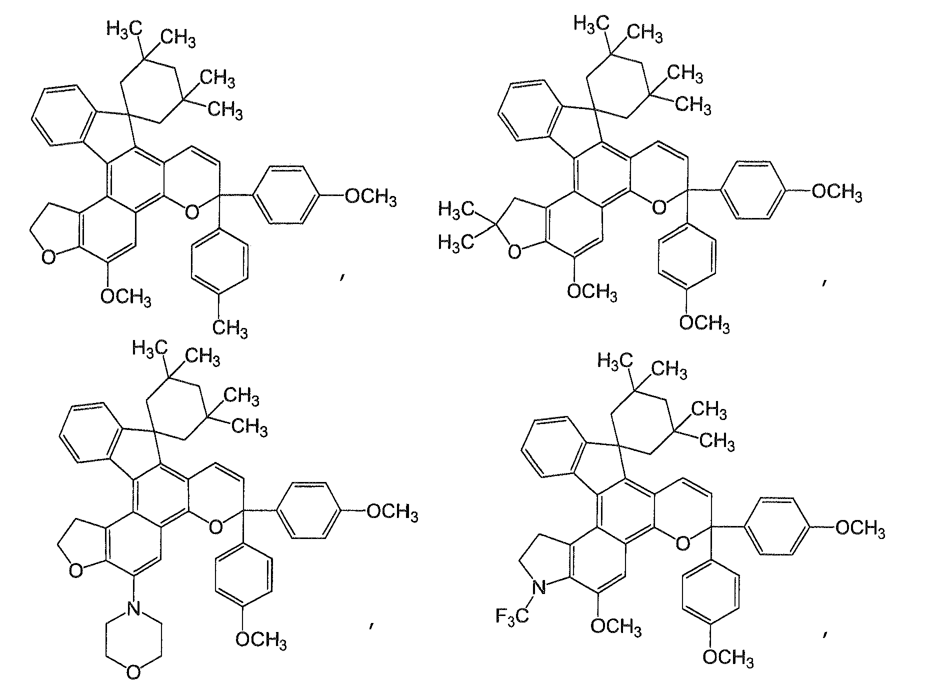

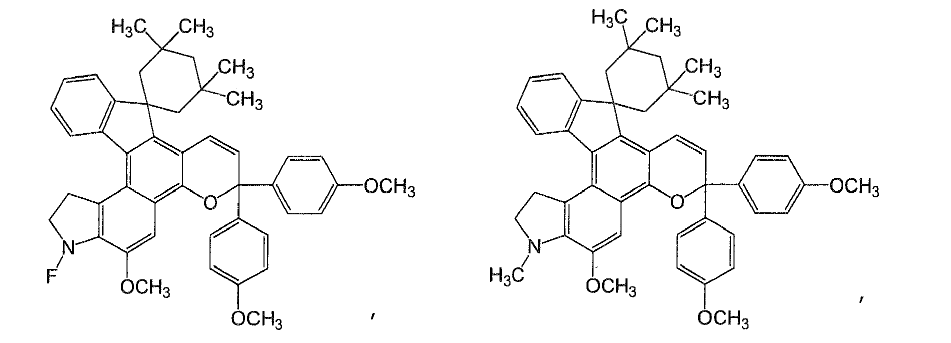

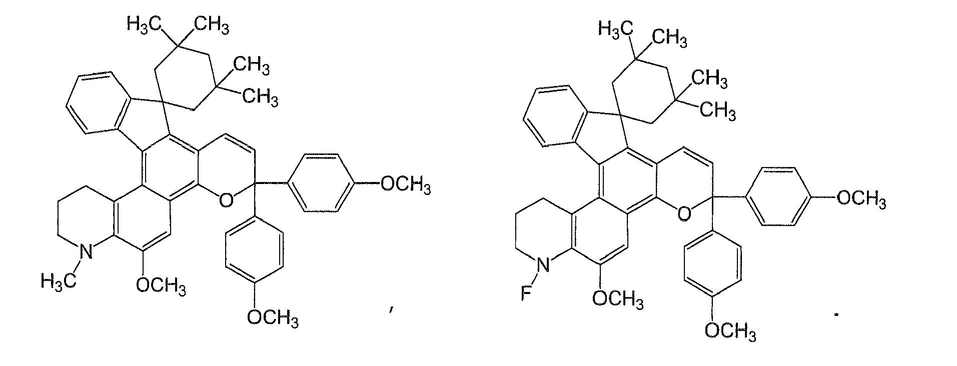

### (identification of chromene compound)

The chromene compound of the present invention is existent as an achromatic or light yellow solid or viscous liquid at normal temperature and normal pressure and can be confirmed by the following means (1) to (3).
(1) When the proton nuclear magnetic resonance spectrum (¹H-NMR) of the chromene compound is measured, a peak based on an aromatic proton appears at δ of around 5.0 to 9.0 ppm and peaks based on the protons of an alkyl group, an alkoxy group and an alkylene group appear at δ of around 0.5 to 4.5 ppm. By comparing these spectral intensities relatively, the number of the protons of bonds can be known.
(2) The composition of a corresponding compound can be determined by elemental analysis.
(3) When the ¹³C-nuclear magnetic resonance spectrum (¹³C-NMR) of the chromene compound is measured, a peak based on the carbon of an aromatic hydrocarbon group appears at δ of around 110 to 160 ppm, a peak based on the carbon of an alkyleneoxy group appears at δ of around 80 to 140 ppm, and peaks based on the carbons of an alkyl group and an alkoxy group appear at δ of around 10 to 80 ppm.

### (production process of chromene compound)

The production process of the chromene compound of the present invention is not particularly limited, and any synthesis process may be employed. As a typical process which is advantageously employed, a corresponding naphthol compound and a corresponding propargyl alcohol compound are reacted with each other. An example of the production process of the chromene compound represented by the above formula (6) will be described hereinbelow.

The chromene compound represented by the above formula (6) can be advantageously produced by reacting a naphthol compound represented by the following formula (8) with a propargyl alcohol represented by the following formula (9) in the presence of an acid catalyst. 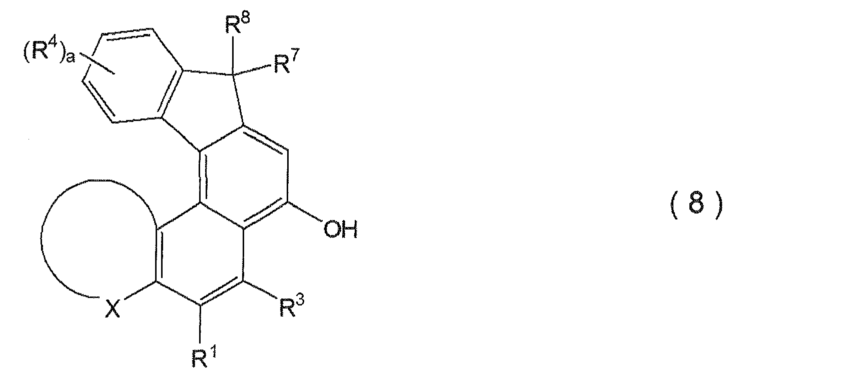 (wherein "a", R¹, R³, R⁴, R⁷, R⁸ and the ring portion including X are as defined in the above formula (6)) 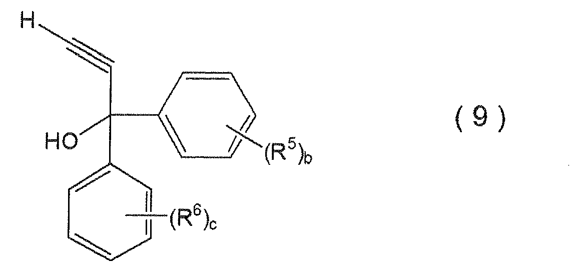 (wherein "b", "c", R⁵ and R⁶ are as defined in the above formula (6))

The naphthol compound represented by the above formula (8) is provided as a novel compound by the present invention. In the formula (8), "a", R¹, R³, R⁴, R⁷, R⁸ and the ring portion including X (same as in the above formula (2)) are as defined in the above formula (6). Therefore, it should be understood that the above explanation of the formula (6) is directly applied to these groups and the portion.

In the present invention, preferred examples of the naphthol compound represented by the above formula (8) are the following compounds.

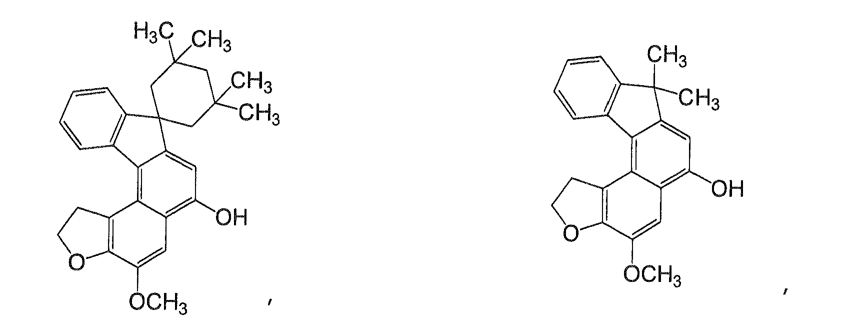

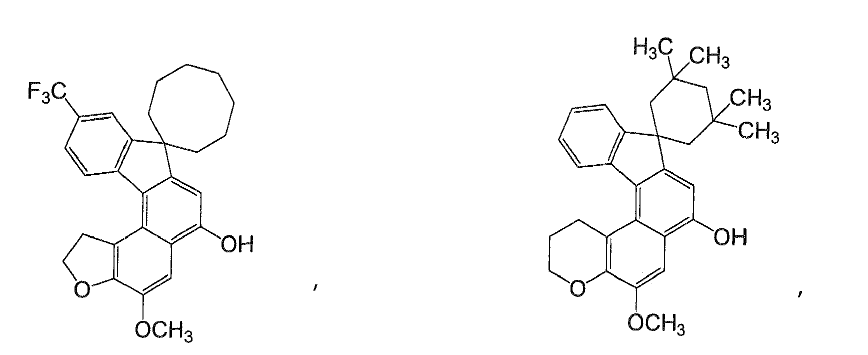

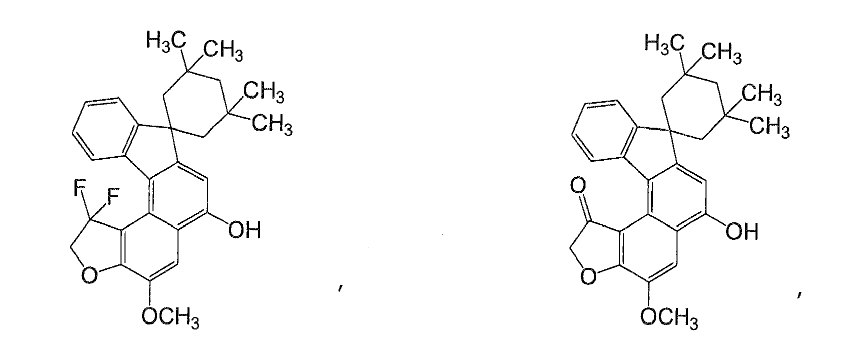

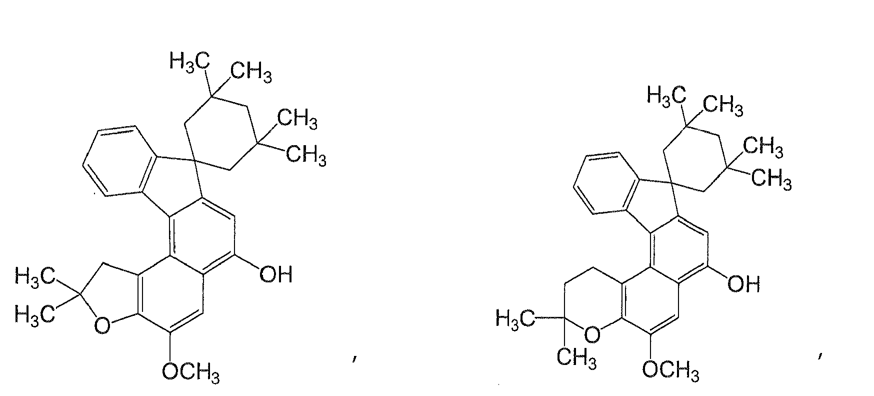

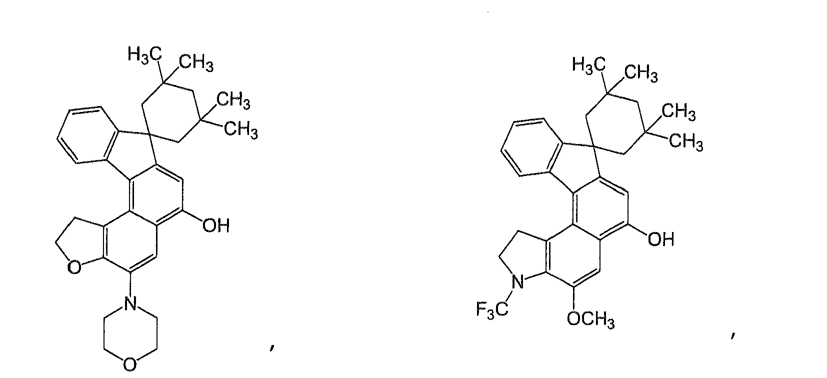

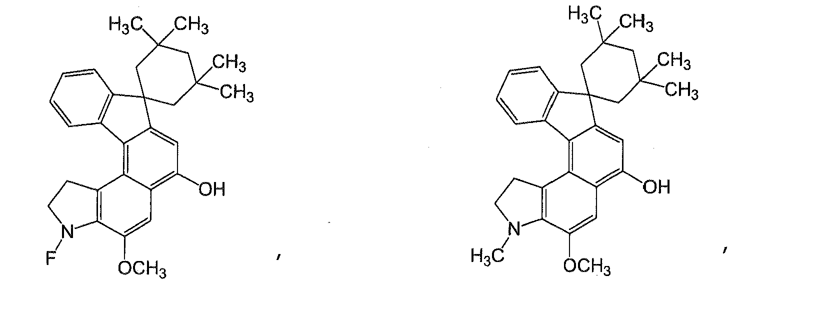

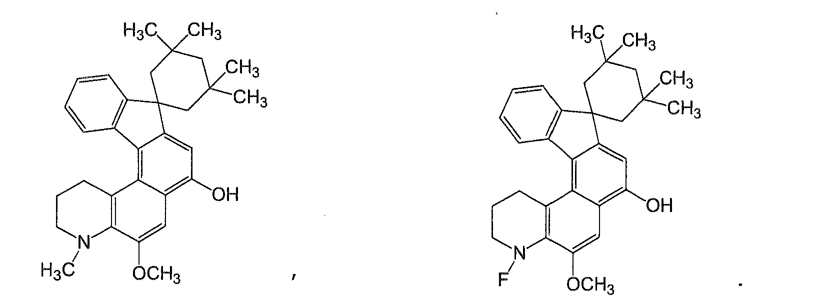

Ordinary naphthol compounds can be synthesized in accordance with a reaction method described in, for example, Journal of Organic Chemistry 69(10)3282-3293; 2004, Synthetic Communications 23(16)2241-2249 (1993) and WO01/60881.

### (process of synthetizing naphthol compound)

Although the process of synthesizing the naphthol compound represented by the above formula (8) is not particularly limited, it can be synthesized as follows, for example.

A benzene compound represented by the following formula (10) may be purchased as a reagent or may be synthesized based on the following documents.

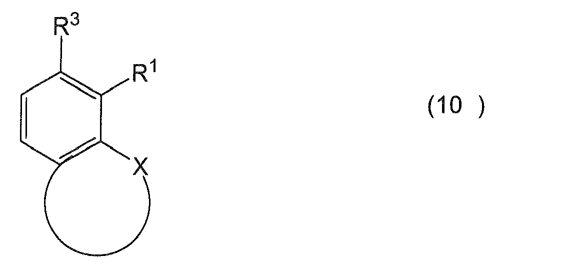

In the above formula, X, R¹ and R³ are as defined in the above formula (2), the above formula (1) and the above formula (6).

For example, a benzene compound represented by the following formula (11) can be synthesized in accordance with a reaction method described in Synthesis. (10). 839-840; 1986 and Journal of Heterocyclic Chemistry, 26(4). 957-964; 1989.

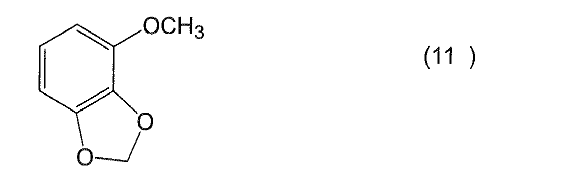

For example, a benzene compound represented by the following formula (12) can be synthesized in accordance with a reaction method described in Synthesis. (15). 2249-2272; 2007, Chemical Communications. (32). 3747-3749; 2008 and Helvetica Chemica Acta. 85. (2). 442-450; 2002.

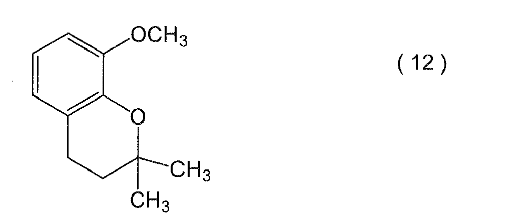

For example, a benzene compound represented by the following formula (13) can be synthesized in accordance with a reaction method described in Tetrahedron Letters, 46 (12). 2071-2074; 2005 and Organic Process Research & Development 5(6). 604-608; 2001.

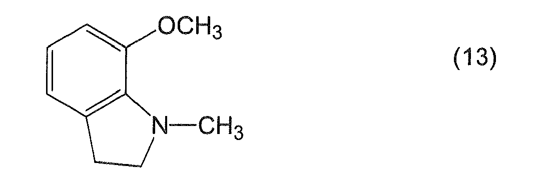

After the obtained benzene compound is brominated, a Grignard reagent is prepared and reacted with acid chloride to obtain a benzophenone compound represented by the following formula (14).

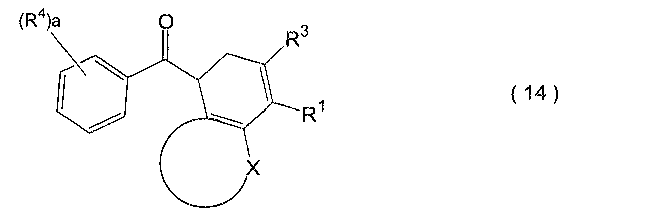

By carrying out the Stobbe reaction and cyclization reaction of the benzophenone compound, a naphthalene compound represented by the following formula (15) is obtained and hydrolyzed by using an alkali or acid to obtain a carboxylic acid represented by the following formula (16).

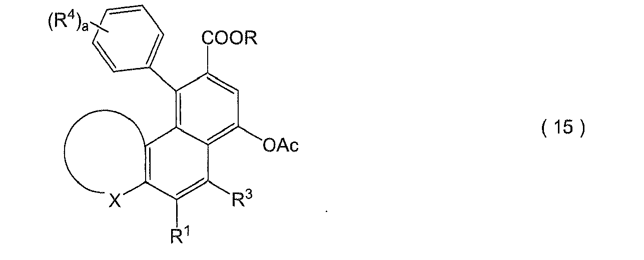

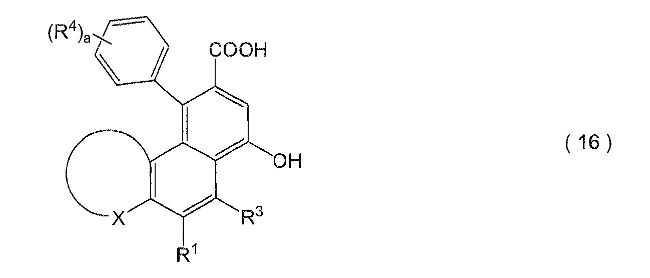

The carboxylic acid is converted into an amine by a method such as Curtius rearrangement, Hofmann rearrangement or Lossen rearrangement, and a diazonium salt is prepared from the amine. This diazonium salt is converted into a bromide by a Sandmeyer reaction or the like, and the obtained bromide is reacted with magnesium or lithium to prepare an organic metal reagent. R⁴ and "a" in the above formulas (14) to (16) are as defined in the above formula (6). This organic metal reagent is reacted with a ketone represented by the following formula (17) at -80 to 70°C in an organic solvent for 10 minutes to 4 hours to obtain an alcohol material represented by the following formula (18). 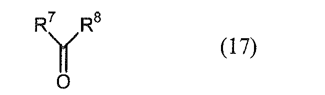 (R⁷ and R⁸ in the above formula (17) are as defined in the above formula (6))

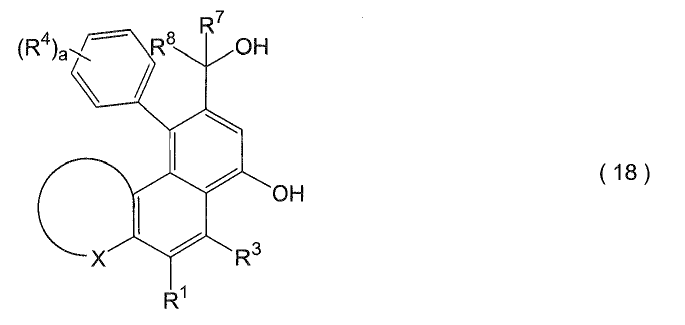

The naphthol compound of interest can be synthesized by carrying out the Friedel-Crafts reaction of this alcohol material at 10 to 120°C for 10 minutes to 2 hours under a neutral to acid condition. In the above reaction, the reaction ratio of the above organic metal reagent to the ketone represented by the above formula (17) is selected from among a wide range but generally selected from a range of 1:10 to 10:1 (molar ratio). The reaction temperature is preferably -80 to 70°C, and an aprotic organic solvent such as diethyl ether, tetrahydrofuran, benzene or toluene is used as the solvent. The naphthol compound represented by the above formula (8) can be obtained by carrying out the Friedel-Crafts reaction of the alcohol material in the neutral to acid condition. The acid catalyst is preferably selected from acetic acid, hydrochloric acid, sulfuric acid, benzenesulfonic acid, p-toluenesulfonic acid and acid alumina. The acid catalyst is preferably used in an amount of 0.1 to 10 parts by weight based on 100 parts by weight of the alcohol material. For this reaction, a solvent such as tetrahydrofuran, benzene or toluene is used.

### (identification of naphthol compound)

The naphthol compound of the present invention is existent as an achromatic or light yellow solid or viscous liquid at normal temperature and normal pressure and can be confirmed by the following means (1) to (3).
(1) When the proton nuclear magnetic resonance spectrum (¹H-NMR) of the naphthol compound is measured, a peak based on an aromatic proton appears at δ of around 5.0 to 9.0 ppm and peaks based on the protons of an alkyl group, an alkoxy group and an alkylene group appear at δ of around 0.5 to 4.5 ppm. By comparing these spectral intensities relatively, the number of the protons of bonds can be known.
(2) The composition of a corresponding compound can be determined by elemental analysis.
(3) When the ¹³C-nuclear magnetic resonance spectrum (¹³C-NMR) of the naphthol compound is measured, a peak based on the carbon of an aromatic hydrocarbon group appears at δ of around 110 to 160 ppm, a peak based on the carbon of an alkyleneoxy group appears at δ of around 80 to 140 ppm, and peaks based on the carbons of an alkyl group and an alkoxy group appear at δ of around 10 to 80 ppm.

### (synthesis of propargyl alcohol)

The propargyl alcohol compound represented by the above formula (9) is an already known compound. The propargyl alcohol compound can be synthesized, for example, by reacting a benzophenone compound corresponding to the above formula (9) with a metal acetylene compound such as lithium acetylide.

### (specific production process of chromene compound)

The reaction ratio of the naphthol compound to the propargyl alcohol compound is selected from among a range of 1:10 to 10:1 (molar ratio). As the acid catalyst is used sulfuric acid, benzenesulfonic acid, p-toluenesulfonic acid or acid alumina. The acid catalyst is used in an amount of 0.1 to 10 pars by weight based on 100 parts by weight of the total of the naphthol compound and the propargyl alcohol compound. The reaction temperature is preferably 0 to 200°C. An aprotic organic solvent such as N-methylpyrrolidone, dimethyl formamide, tetrahydrofuran, benzene or toluene is preferably used as the solvent. The method of purifying the product obtained through the above reaction is not particularly limited. For example, the obtained product may be purified by carrying out silica gel column purification and further recrystallization.

### (characteristic properties of chromene compound)

Since the chromene compound of the present invention has high double peak characteristic, when it is mixed with another photochromic compound to prepare a photochromic composition which develops a brown or grey color, the amount of the photochromic compound which develops a yellow color and has low durability can be reduced, and a color change at the time of fading and a color change at the time of deterioration hardly occur. Further, since the chromene compound has little initial coloration, an optical article containing the chromene compound of the present invention, for example, a photochromic lens containing the chromene compound of the present invention has high transparency under no exposure to light.

The photochromic compound to be mixed with the chromene compound of the present invention so as to adjust the color is a known compound. Examples of this photochromic compound include chromene compounds described in WO01/060811 and JP-A 2009-67680.

### (use purpose of chromene compound)

The chromene compound of the present invention exhibits excellent photochromic properties as described above. A polymer material dispersion of the chromene compound of the present invention is most practical, and a photochromic optical article having a polymer molded product containing the chromene compound of the present invention dispersed therein as a constituent member exhibits excellent photochromic properties. Therefore, the chromene compound of the present invention can be used especially in photochromic lenses (optical articles).

When the chromene compound of the present invention is used in a photochromic lens, a lens is obtained by a method known per se, capable of obtaining uniform light controllability. For example, a method in which a thermoplastic resin and the chromene compound of the present invention are mixed together in a molten state to form a lens is employed. Further, a method in which a polymer film containing the chromene compound of the present invention dispersed uniformly therein is formed on the surface of a lens, or a method in which the chromene compound of the present invention is dissolved, for example, in silicone oil and impregnated into the surface of a lens at 150 to 200°C for 10 to 60 minutes is also employed. When the chromene compound is dispersed into the surface portion of the lens as described above, the surface of the lens may be further coated with a curable substance as required to obtain a photochromic lens.

Moreover, a method in which a photochromic curable composition containing the chromene compound of the present invention and a polymerizable monomer is polymerized by a predetermined method to obtain a lens may be employed. In the method using this photochromic curable composition, a photochromic lens can be formed directly by polymerizing the curable composition by a known method. Further, the photochromic curable composition may be applied to a plastic lens (optical substrate) and polymerized and cured to form a polymer film containing the chromene compound of the present invention dispersed therein on the optical substrate, thereby obtaining a photochromic lens (optical article) (this method may be referred to as "coating method"). When the polymer film containing the chromene compound of the present invention dispersed therein is formed on the optical substrate, the surface of the polymer film may be further coated with a curable substance.

In the above photochromic curable composition, the polymerizable monomer in use is not particularly limited, known polymerizable monomers may be used, and a combination of known polymerizable monomers may be selected according to the desired performance of an optical article.

### Examples

The following examples are provided for the purpose of further illustrating the present invention but are in no way to be taken as limiting.

### Example 1

1.00 g (2.33 mmol) of a naphthol compound represented by the following formula (19) and 0.92 g (3.43 mmol) of a propargyl alcohol compound represented by the following formula (20) were dissolved in 50 ml of toluene, 0.022 g of p-toluenesulfonic acid was further added, and the obtained mixture was refluxed for 1 hour. 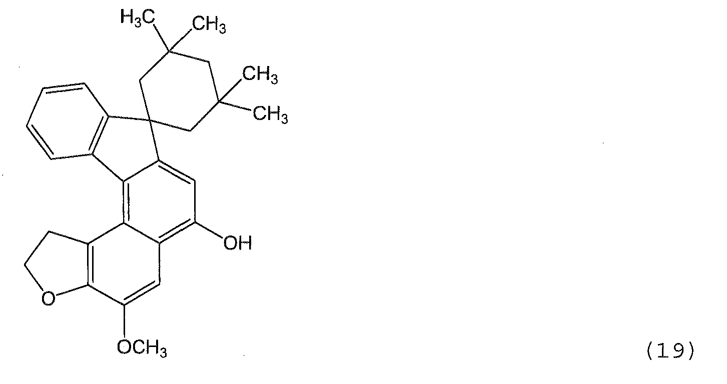 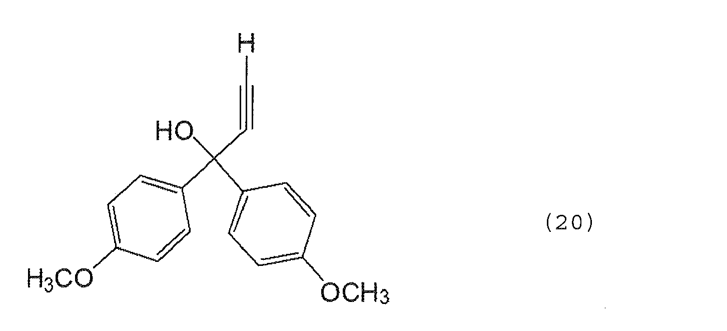 After the reaction, the solvent was removed, the obtained product was purified by column chromatography, and crystallization was carried out with methanol (5 ml) to obtain 1.10 g of a white powder (yield rate of 70 %). The elemental analysis values of this product were 81.31 % of C, 6.89 % of H and 0.00 % of N which were almost equal to the calculated values of C₄₆H₄₆O₅ (C: 81.39 %, H: 6.83 %, N: 0.00 %).

When the proton nuclear magnetic resonance spectrum of the product was measured, it showed 31H peaks based on an alkyl group and an alkoxy group at δ of around 0.5 to 4.5 ppm, and a 15H peak based on an aromatic proton at δ of around 5.0 to 9.0 ppm.

Further, when the ¹³C-nuclear magnetic resonance spectrum was measured, it showed a peak based on the carbon of an aromatic ring at δ of around 110 to 160 ppm, a peak based on the carbon of an alkyleneoxy group at δ of around 80 to 140 ppm and peaks based on the carbons of an alkyl group and an alkoxy group at δ of around 20 to 80 ppm.

It was confirmed from the above results that the isolated product was a compound represented by the following formula (21).

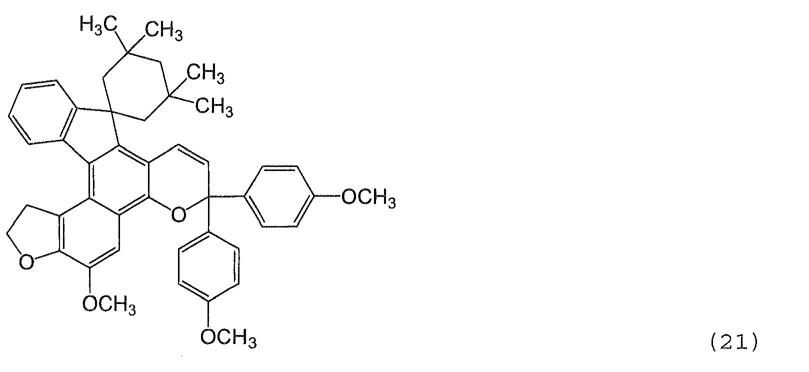

### Examples 2 to 5

Chromene compounds shown in Table 1 were synthesized in the same manner as in Example 1. When the structures of the obtained products were analyzed by using the same structure checking means as in Example 1, it was confirmed that they were compounds represented by structural formulas shown in Table 1. The elemental analysis values of these compounds and the calculated values and characteristic ¹H-NMR spectra obtained from the structural formulas of the compounds are shown in Table 2. The compound Nos. 2 to 5 in Table 2 are chromene compounds obtained in Examples 2 to 5, respectively.

**Table 1**

| Ex. No. | Raw materials | | Product | Yield rate (%) |
|---|---|---|---|---|
| | Naphthol compound | Propargyl alcohol compound | | |
| 2 | 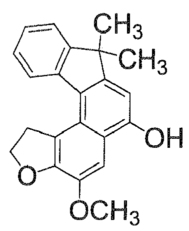 | 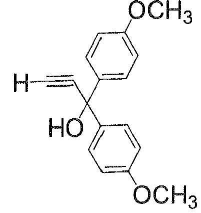 | 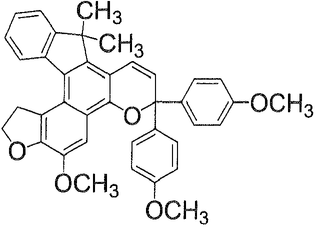 | 65 |
| 3 | 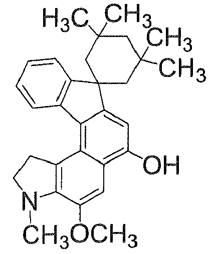 | 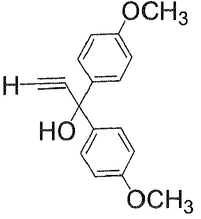 | 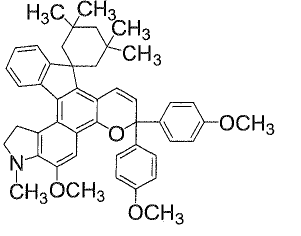 | 67 |
| 4 | 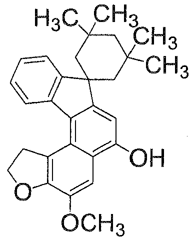 | 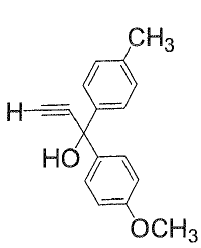 | 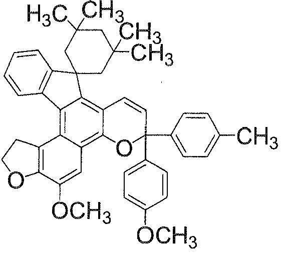 | 72 |
| 5 | 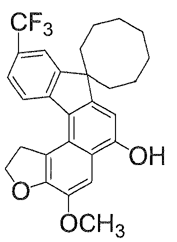 | 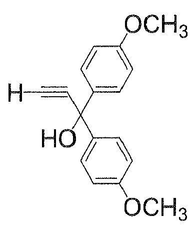 | 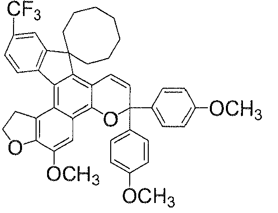 | 73 |

**Table 2**

| Compound No. | Elemental analysis values | | | | | | ¹H-NMR (NMR) |
|---|---|---|---|---|---|---|---|
| | Experimental value | | | Calculated value | | | |
| | C | H | N | C | H | N | |
| 2 | 80.28 | 5.89 | 0.00 | 80.39 | 5.88 | 0.00 | δ5.0-9.0 15H |
| | | | | | | | δ0.5-4.5 19H |
| 3 | 81.48 | 7.16 | 2.22 | 81.59 | 7.14 | 2.02 | δ5.0-9.0 15H |
| | | | | | | | 60.5-4.S 24H |
| 4 | 83.19 | 7.09 | 0.00 | 83.35 | 6.99 | 0.00 | δ5.0-9.0 15H |
| | | | | | | | δ0.5-4.5 25H |
| 5 | 75.29 | 5.75 | 0.00 | 75.29 | 5.75 | 0.00 | δ5.0-9.0 14H |
| | | | | | | | δ0.5-4.5 27H |

### Example 6

### (evaluation of physical properties of photochromic plastic lens produced by coating method)

The chromene compound obtained in the above example was mixed with a photopolymerization initiator and a polymerizable monomer, the resulting mixture was applied to the surface of a lens substrate, and ultraviolet light was applied to polymerize the coating film on the surface of the lens substrate.

A photochromic curable composition prepared by mixing together 50 parts by mass of 2,2-bis(4-methacryloyloxypentaethoxyphenyl)propane, 10 parts by mass of polyethylene glycol diacrylate (average molecular weight of 532), 10 parts by mass of trimethylolpropane trimethacrylate, 10 parts by mass of polyester oligomer hexaacrylate (EB-1830 of Daicel UCB Co., Ltd.) and 10 parts by mass of glycidyl methacrylate was used as radical polymerizable monomers. 1 part by mass of the chromene compound obtained in Example 1 was added to and fully mixed with 90 parts by mass of this mixture of the radical polymerizable monomers, and 0.3 part by mass of CGI1800 {a mixture of 1-hydroxycyclohexylphenyl ketone and bis(2,6-dimethoxybenzoyl)-2,4,4-trimethyl-pentyl phosphine oxide (weight ratio of 3:1) as a photopolymerization initiator, 5 parts by mass of bis(1,2,2,6,6-pentamethyl-4-piperidyl)sebacate and 3 parts by mass of ethylenebis(oxyethylene)bis[3-(5-tert-butyl-4-hydroxy-m-tolyl)propionate] as a stabilizer, 7 parts by mass of y-methacryloyloxypropyl trimethoxysilane as a silane coupling agent and 3 parts by mass of N-methyldimethanolamine were added to and fully mixed with the above mixture to obtain a photochromic curable composition.

Subsequently, about 2 g of the photochromic curable composition obtained by the above method was applied to the surface of a lens substrate (CR39: acrylic resin plastic lens; refractive index of 1.50) by using the 1H-DX2 spin coater of MIKASA Co., Ltd. This coated lens was irradiated with light of a metal halide lamp having an output of 120 mW/cm² in a nitrogen gas atmosphere for 3 minutes to cure the photochromic curable composition so as to produce an optical article coated with a polymer film containing the chromene compound dispersed therein (thickness of polymer film: 40 µm) (photochromic plastic lens).

The following photochromic properties of the obtained photochromic plastic lens were evaluated.
[1] Maximum absorption wavelength (λmax) : This is the maximum absorption wavelength after color development obtained by means of the spectrophotometer (MCPD3000 instantaneous multi-channel photodetector) of Otsuka Electronics Co., Ltd.) and used as an index of color at the time of color development.
[2] Color optical density (A₀) : This is the difference between absorbance {ε(120) after 120 seconds of exposure and ε (0) at the above maximum absorption wavelength and used as an index of color optical density. As this value becomes larger, the photochromic properties become better.
[3] Double peak characteristic (Ay/A_{B}) : This is the ratio of color optical density (Ay: value of λₘₐₓ) at a yellow range (430 to 530 nm) and color optical density (A_{B}: value of λₘₐₓ) at a blue range (550 to 650 nm) and used as an index of double peak characteristic.
[4] Fading half period [τ1/2(sec.)]: time required for the reduction of the absorbance at the above maximum absorption wavelength of a sample to 1/2 of {ε(120)-ε(0)} when exposure is stopped after 120 seconds of exposure and used as an index of fading speed. As this time becomes shorter, the fading speed becomes higher.
[5] Absorption end {λ₀} : After the photochromic plastic lens obtained under the above conditions is used as a sample and kept in the dark for one day, the transmittance (T%) at 300 to 800 nm of the sample is measured with an ultraviolet visible spectrophotometer (UV-2550 of Shimadzu Corporation) at room temperature. A tangent line is drawn on the obtained transmittance curve to ensure that the transmittance (T%) of the transmittance curve passes a point of 50 % so as to obtain a wavelength at which the transmittance (T%) of the tangent line becomes 0 as the absorption end (absorption end of the spectrum) and used as an index of initial coloration. For example, in an optical article such as a spectacle lens, as this value becomes smaller, the initial coloration becomes weaker and the transparency under no irradiation becomes higher.
[6] Thermochromism {T₀} : The photochromic plastic lens obtained under the above conditions is used as a sample and its transmittance (T%) at 300 to 800 nm is measured with an ultraviolet visible spectrophotometer (UV-2550 of Shimadzu Corporation) at room temperature. This is a transmittance at a wavelength at which the transmittance at 430 to 650 nm becomes minimal and used as an index of initial coloration. As this value becomes larger, the initial coloration becomes weaker and the transparency under no irradiation becomes higher.
[7] Residual rate (A₅₀/A₀ x 100) : The deterioration promotion test of the obtained photochromic plastic lens is carried out by using the X25 xenon weather meter of Suga Test Instruments Co., Ltd. for 50 hours. Thereafter, the above color optical density is evaluated before and after the test, the color optical density (A₀) before the test and the color optical density (A₅₀) after the test are measured, and the ratio (A₅₀/A₀) of these values is taken as residual rate and used as an index of color development durability. As the residual rate becomes higher, the color development durability becomes higher.

### Examples 7 to 10

Photochromic plastic lenses were manufactured and their characteristic properties were evaluated in the same manner as in Example 6 except that the compounds obtained in Examples 2 to 5 were used as the chromene compound. The results are shown in Table 3. In Table 3, the compound Nos. 1 to 5 are chromene compounds obtained in Examples 1 to 5, respectively.

**Table 3**

| Example No. | Compound No. | λ max | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | A_{50/}A₀) × 100 |
| 6 | 1 | 446 | 0.50 | 1.52 | 67 | 403 | 87 | 88 |
| | | 567 | 0.33 | | 68 | | 88 | 89 |
| 7 | 2 | 450 | 0.57 | 1.50 | 148 | 404 | 80 | 88 |
| | | 569 | 0.38 | | 152 | | 81 | 89 |
| 8 | 3 | 462 | 0.62 | 1.82 | 95 | 410 | 83 | 83 |
| | | 563 | 0.34 | | 96 | | 84 | 84 |
| 9 | 4 | 436 | 0.62 | 1.51 | 91 | 403 | 87 | 85 |
| | | 557 | 0.41 | | 92 | | 88 | 86 |
| 10 | 5 | 448 | 0.37 | 1.54 | 96 | 403 | 84 | 83 |
| | | 569 | 0.24 | | 98 | | 85 | 84 |

### Comparative Examples 1 to 8

For comparison, the operation of Example 6 was repeated by using the compound of the following formula (A) (Comparative Example 1), the compound of the following formula (B) (Comparative Example 2), the compound of the following formula (C) (Comparative Example 3), the compound of the following formula (D) (Comparative Example 4), the compound of the following formula (E) (Comparative Example 5), the compound of the following formula (F) (Comparative Example 6), the compound of the following formula (G) (Comparative Example 7) and the compound of the following formula (H) (Comparative Example 8). The chromene compounds used in these comparative examples are given below.

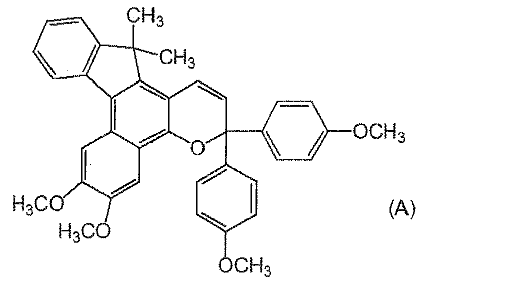

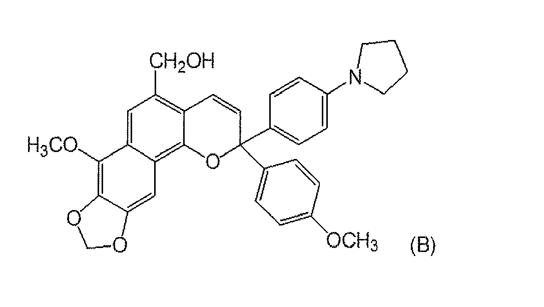

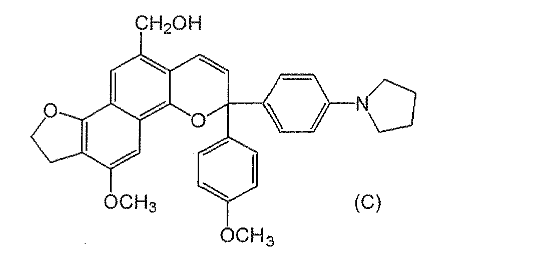

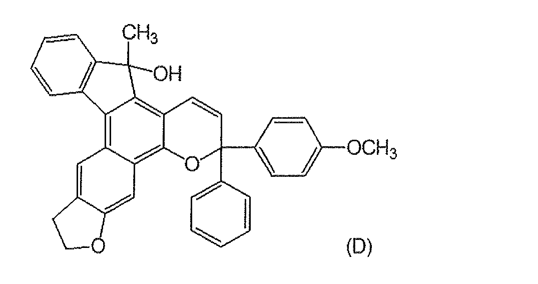

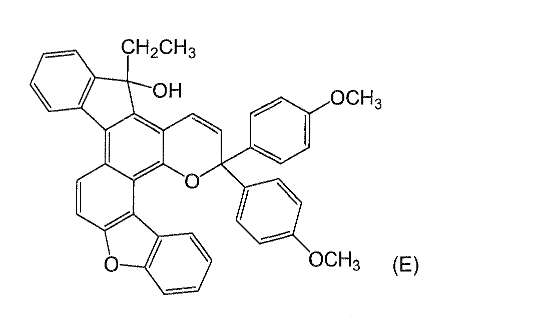

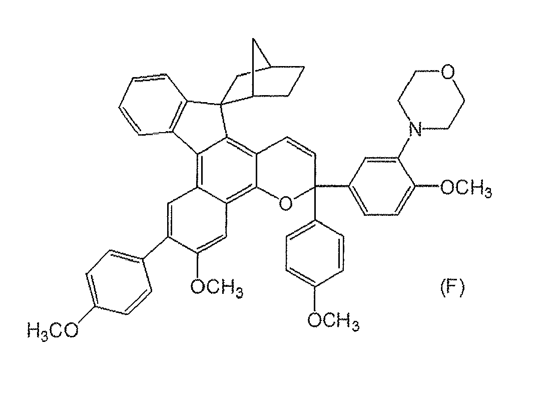

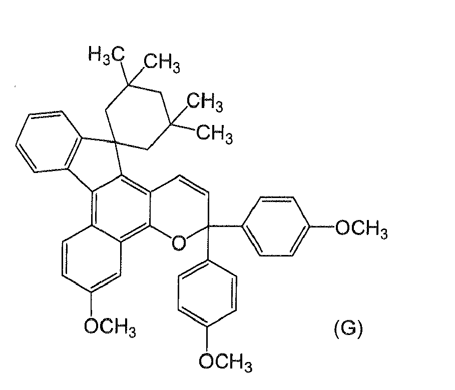

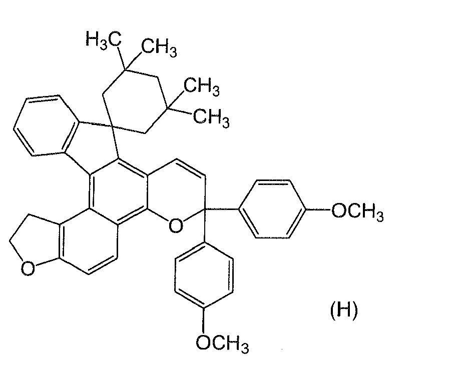

Photochromic plastic lenses were obtained by using the above chromene compounds and their photochromic properties were evaluated in the same manner as in Example 6. The results are shown in Table 4.

**Table 4**

| Comparative Example No. | Compound No. | λmax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} | τ1/2 (sec) | (nm) | (%) | (A₅₀/A₀) X100 |
| 1 | A | 457 | 0.69 | 1.56 | 195 | 397 | 67 | 76 |
| | | 574 | 0.45 | | 196 | | 75 | 77 |
| 2 | B | 475 | 0.26 | 0.80 | 140 | 404 | 77 | 69 |
| | | 585 | 0.32 | | 140 | | 77 | 69 |
| 3 | C | 470 | 0.25 | 0.64 | 116 | 411 | 78 | 68 |
| | | 580 | 0.39 | | 116 | | 78 | 68 |
| 4 | D | 455 | 0.30 | 0.94 | 83 | 410 | 77 | 35 |
| | | 576 | 0.32 | | 83 | | 78 | 35 |
| 5 | E | 485 | 0.28 | 0.84 | 83 | 418 | 74 | 69 |
| | | 595 | 0.37 | | 83 | | 74 | 70 |
| 6 | F | 458 | 0.44 | 1.20 | 68 | 422 | 84 | 85 |
| | | 568 | 0.37 | | 68 | | 86 | 84 |
| 7 | G | 439 | 0.33 | 0.80 | 57 | 419 | 88 | 86 |
| | | 551 | 0.41 | | 57 | | 88 | 86 |
| 8 | H | 441 | 0.32 | 0.71 | 51 | 382 | 87 | 86 |
| | | 550 | 0.40 | | 52 | | 87 | 87 |

In Comparative Example 1, although color optical density and double peak characteristic are satisfactory, initial coloration due to thermochromism is large and the fading speed is low. In Comparative Examples 2, 3, 4, 5, 7 and 8, since the double peak characteristic is low, color control is not satisfactory when a photochromic plastic lens having a color of a neutral tint is to be manufactured. In Comparative Example 6, although color optical density and double peak characteristic are satisfactory, initial coloration is large as the absorption end goes beyond 420 nm into a visible range.

In contrast to this, in Examples in which the chromene compound of the present invention is used, as compared with Comparative Example 1, initial coloration due to thermochromism is little and the fading speed is high. As compared with Comparative Examples 2, 3, 4, 5, 7 and 8, double peak characteristic is high. Since the absorption end is at a short wavelength as compared with Comparative Example 6, initial coloration is little. Further, the chromene compounds of the present invention are all satisfactory in terms of durability.

### Examples 11 to 51

Chromene compounds shown in Table 5 were synthesized in the same manner as in Example 1. When the structures of the obtained chromene compounds were analyzed in the same manner as in Example 1, it was confirmed that they were compounds represented by the structural formulas shown in Table 5. Table 6 shows the elemental analysis values and ¹H spectral values of the chromene compounds obtained in Examples. In Table 6, the compound Nos. 11 to 51 are chromene compounds obtained in Examples 11 to 51, respectively.

**Table 5**

| Ex. No. | Raw materials | | Product | Yield rate (%) |
|---|---|---|---|---|
| | Naphthol compound | Propargyl alcohol | | |
| 11 | 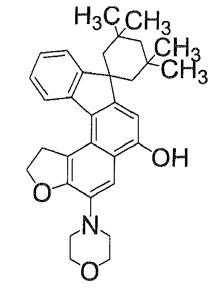 | 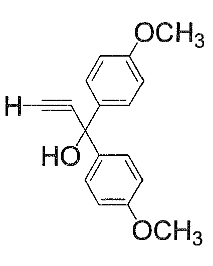 | 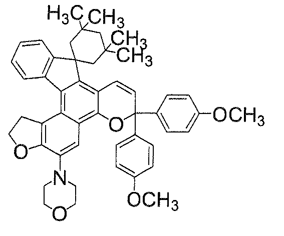 | 64 |
| 12 | 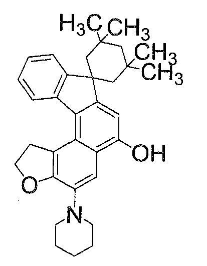 | 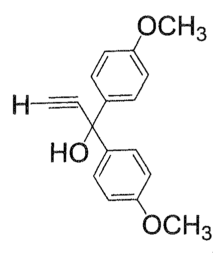 | 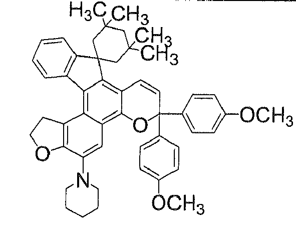 | 65 |
| 13 | 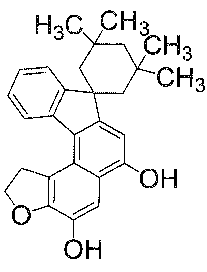 | 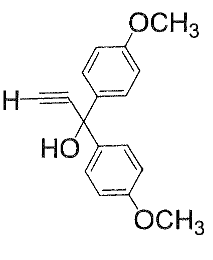 | 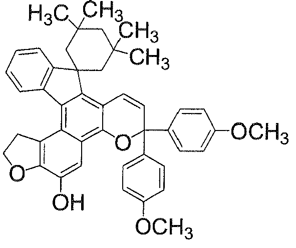 | 59 |
| 14 | 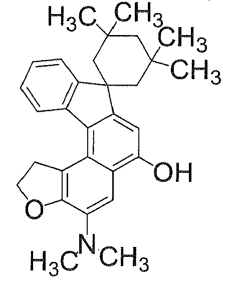 | 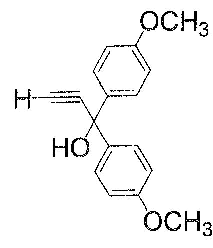 | 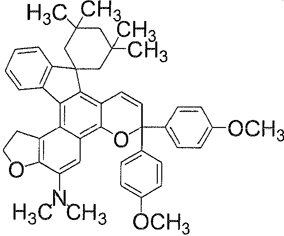 | 62 |
| 15 | 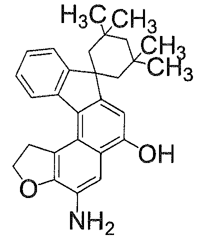 | 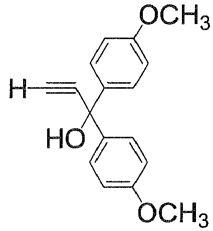 | 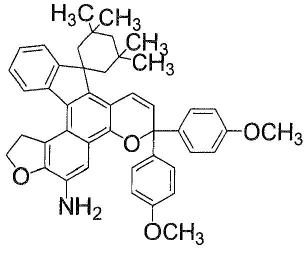 | 62 |
| 16 | 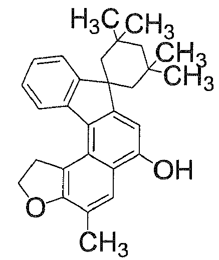 | 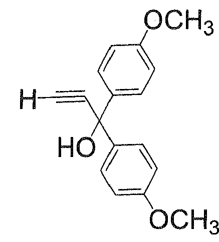 | 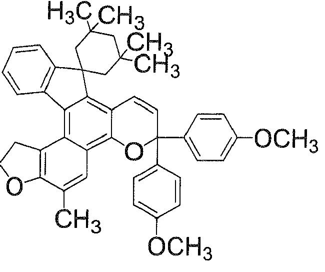 | 75 |
| 17 | 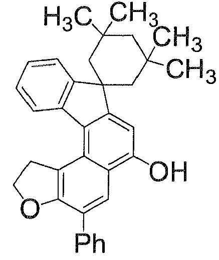 | 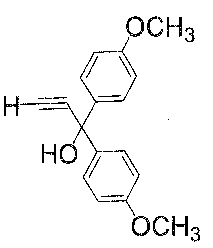 | 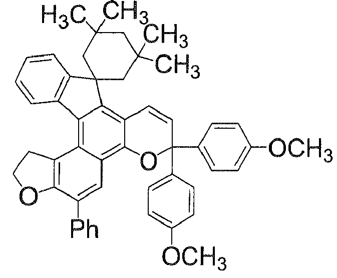 | 77 |
| 18 | 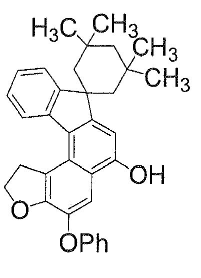 | 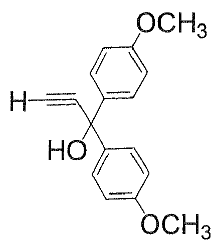 | 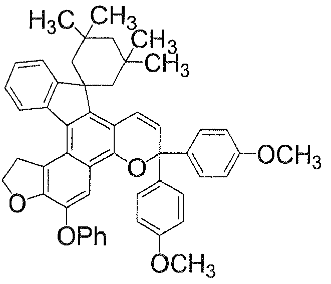 | 72 |
| 19 | 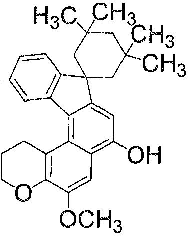 | 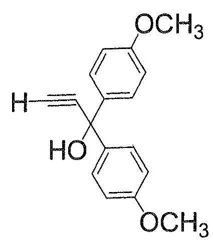 | 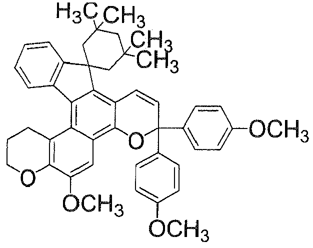 | 75 |
| 20 | 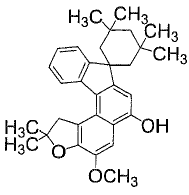 | 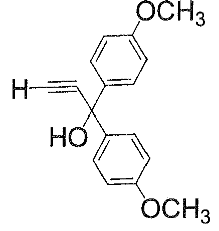 | 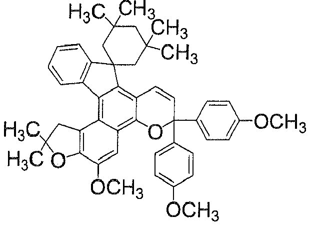 | 77 |
| 21 | 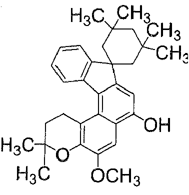 | 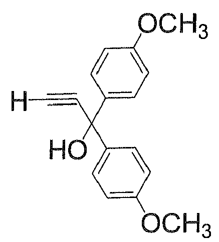 | 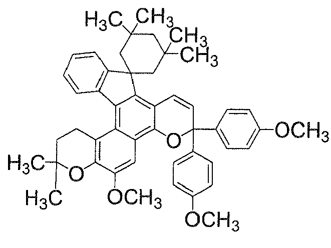 | 72 |
| 22 | 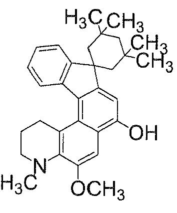 | 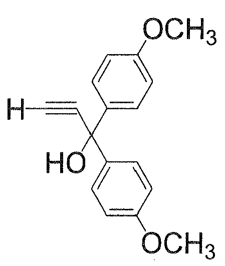 | 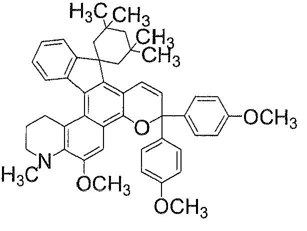 | 73 |
| 23 | 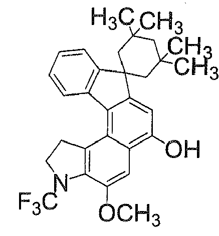 | 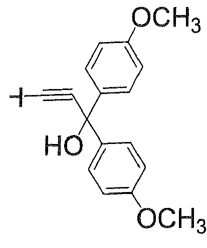 | 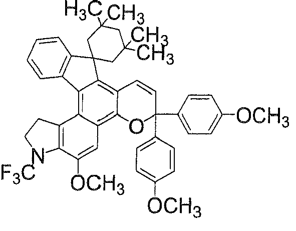 | 65 |
| 24 | 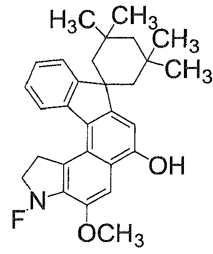 | 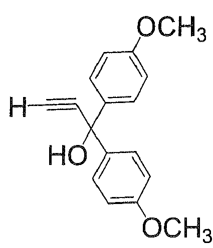 | 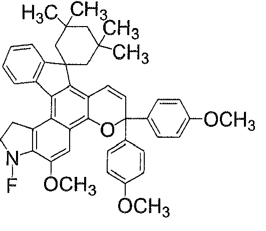 | 67 |
| 25 | 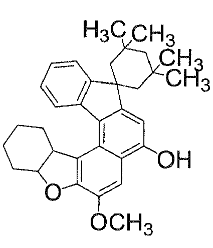 | 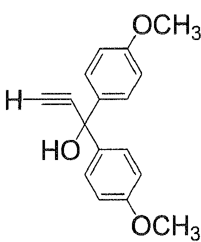 | 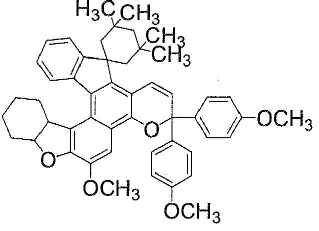 | 73 |
| 26 | 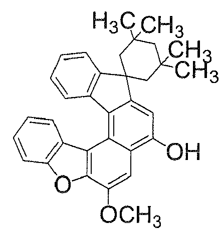 | 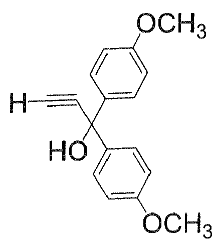 | 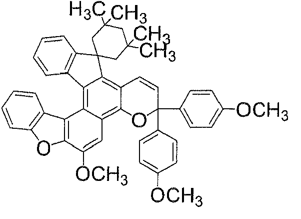 | 71 |
| 27 | 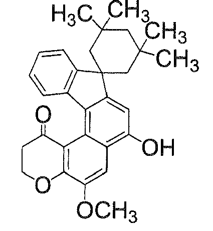 | 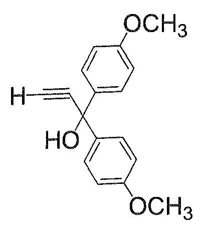 | 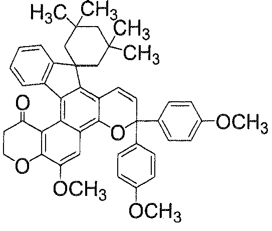 | 67 |
| 28 | 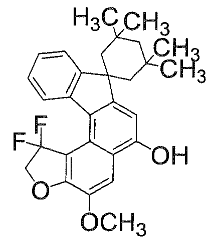 | 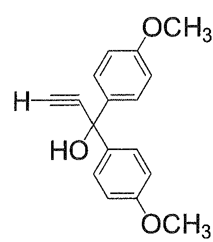 | 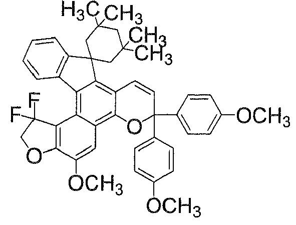 | 70 |
| 29 | 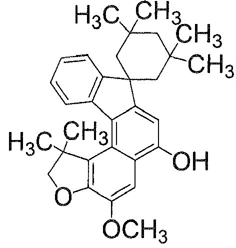 | 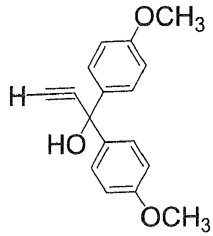 | 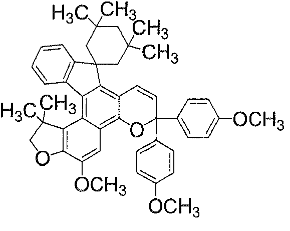 | 69 |
| 30 | 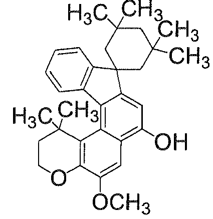 | 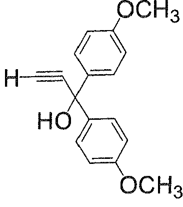 | 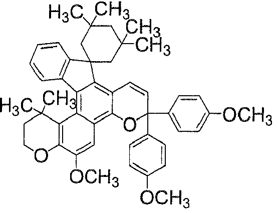 | 72 |
| 31 | 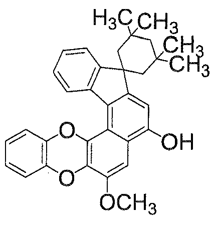 | 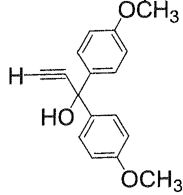 | 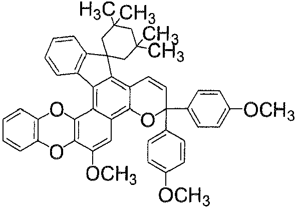 | 65 |
| 32 | 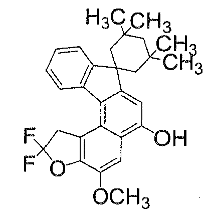 | 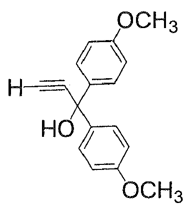 | 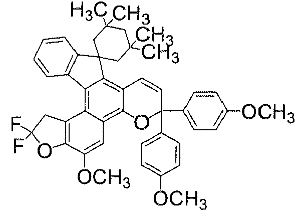 | 64 |
| 33 | 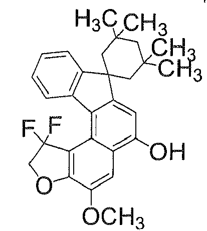 | 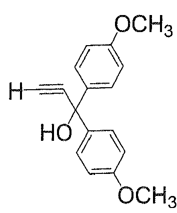 | 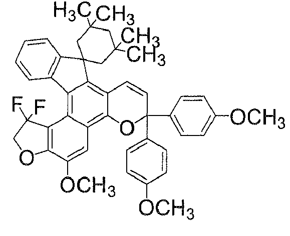 | 67 |
| 34 | 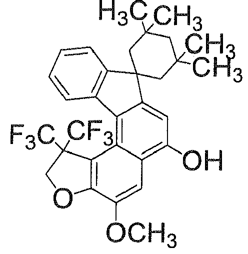 | 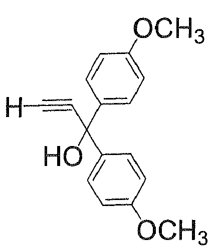 | 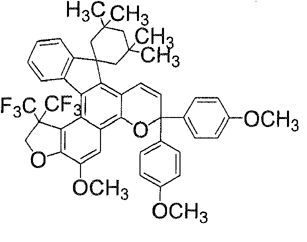 | 65 |
| 35 | 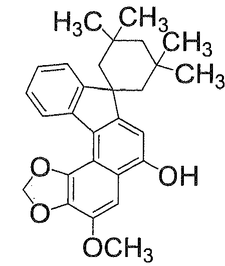 | 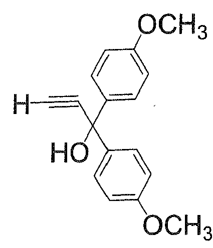 | 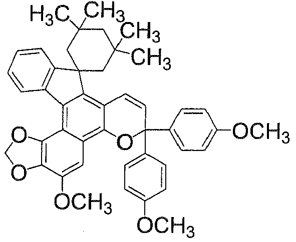 | 71 |
| 36 | 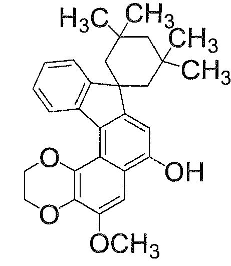 | 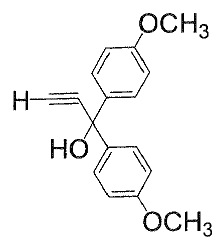 | 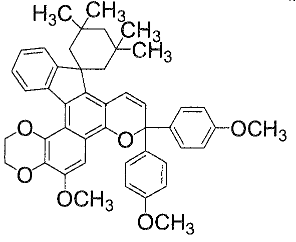 | 72 |
| 37 | 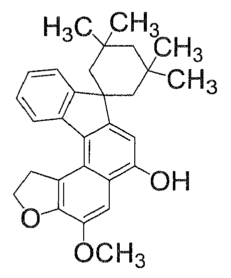 | 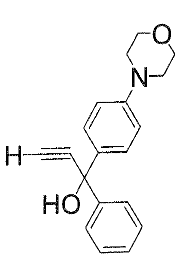 | 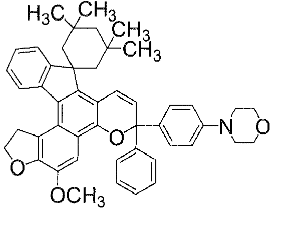 | 66 |
| 38 | 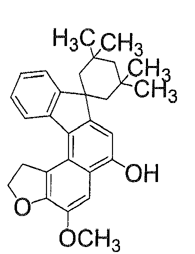 | 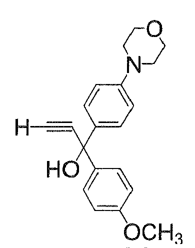 | 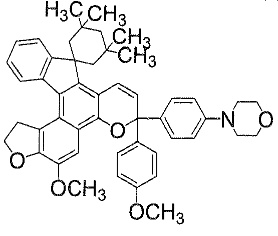 | 63 |
| 39 | 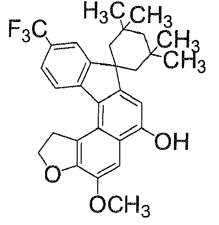 | 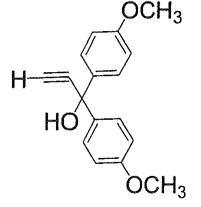 | 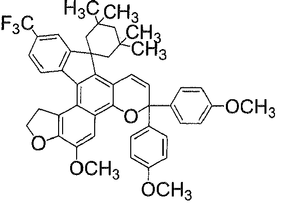 | 65 |
| 40 | 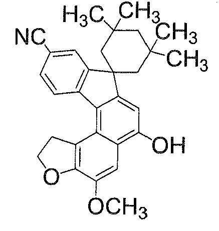 | 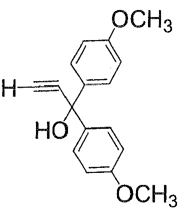 | 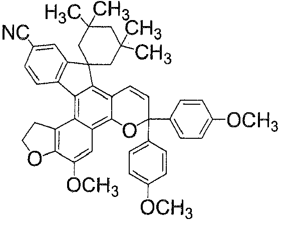 | 67 |
| 41 | 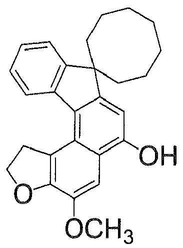 | 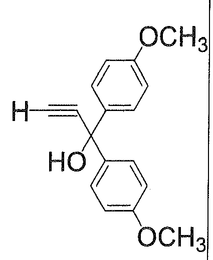 | 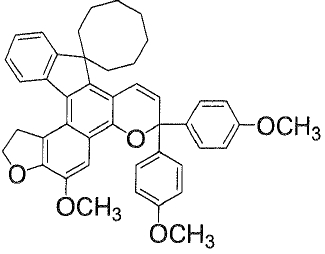 | 73 |
| 42 | 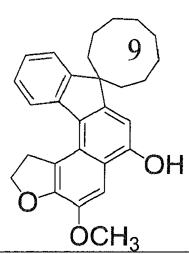 | 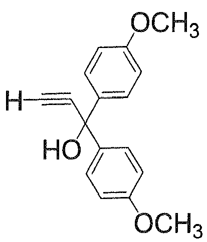 | 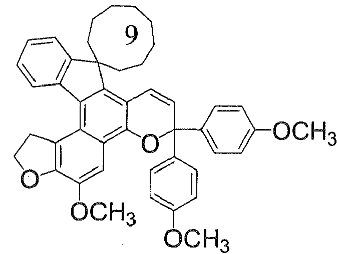 | 73 |
| 43 | 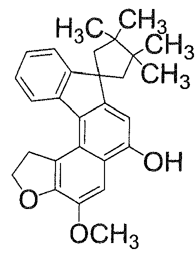 | 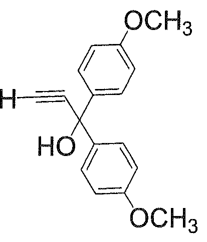 | 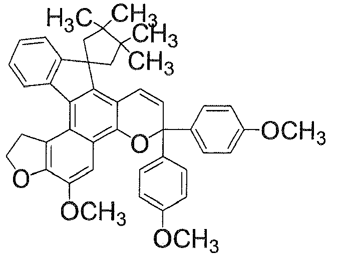 | 69 |
| 44 | 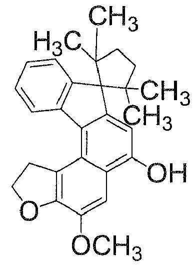 | 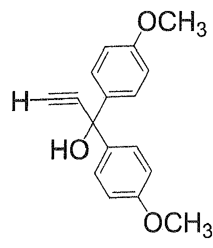 | 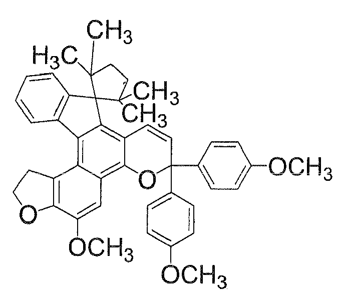 | 69 |
| 45 | 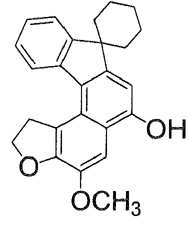 | 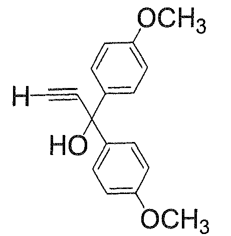 | 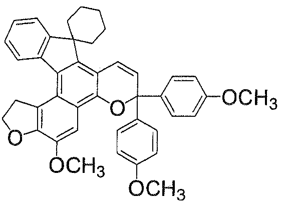 | 70 |
| 46 | 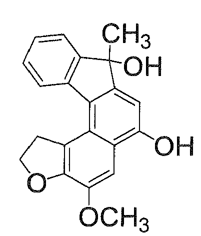 | 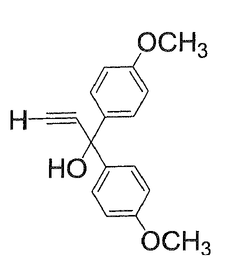 | 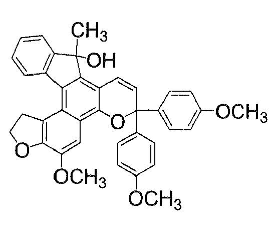 | 65 |
| 47 | 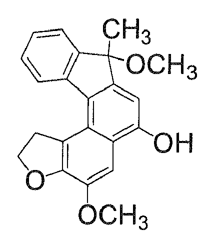 | 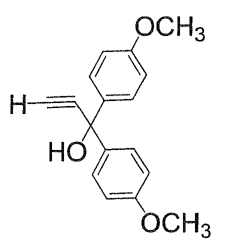 | 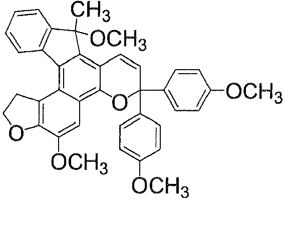 | 66 |
| 48 | 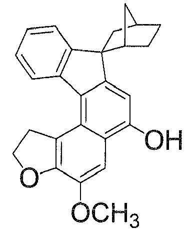 | 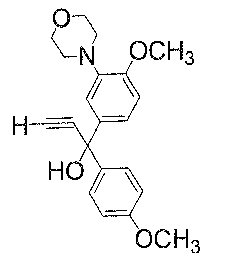 | 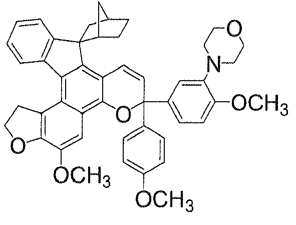 | 62 |
| 49 | 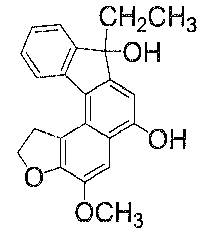 | 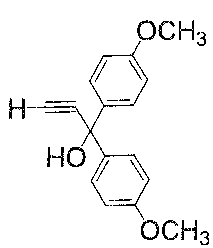 | 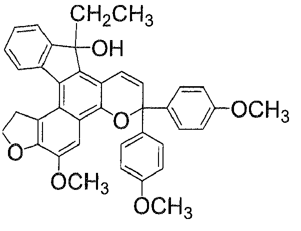 | 72 |
| 50 | 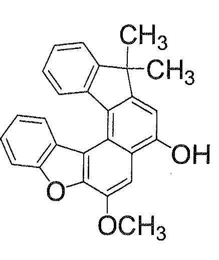 | 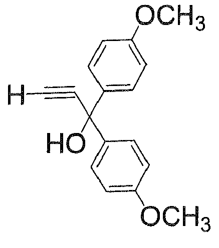 | 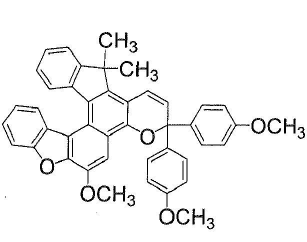 | 69 |
| 51 | 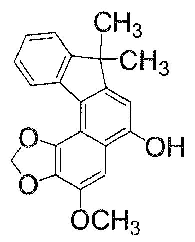 | 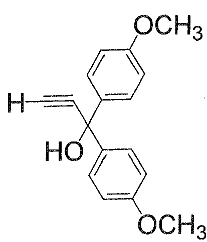 | 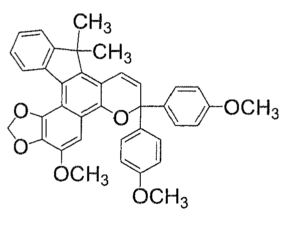 | 70 |

**Table 6**

| Compound No. | Elemental analysis values | | | | | | ¹H-NMR (NMR) |
|---|---|---|---|---|---|---|---|
| | Experimental values | | | Calculated values | | | |
| | C | H | N | C | H | N | |
| 11 | 80.23 | 7.05 | 1.85 | 80.19 | 7.00 | 1.91 | δ5.0-9.0 15H |
| | | | | | | | δ0.5-4.5 36H |
| 12 | 82.00 | 7.26 | 1.92 | 82.04 | 7.30 | 1.92 | δ5.0-9.0 15H |
| | | | | | | | δ0.5-4.5 38H |
| 13 | 81.37 | 6.65 | 0.00 | 81.30 | 6.67 | 0.00 | δ5.0-9.0 16H |
| | | | | | | | δ0.5-4.5 28H |
| 14 | 81.66 | 7.11 | 1.94 | 81.59 | 7.14 | 2.02 | δ5.0-9.0 15H |
| | | | | | | | δ0.5-4.5 34H |
| 15 | 81.44 | 6.89 | 2.11 | 81.42 | 6.83 | 2.11 | δ5.0-9.0 15H |
| | | | | | | | δ0.5-4.5 30H |
| 16 | 83.43 | 7.03 | 0.00 | 83.35 | 6.99 | 0.00 | δ5.0-9.0 15H |
| | | | | | | | δ0.5-4.5 31H |
| 17 | 84.59 | 6.72 | 0.00 | 84.50 | 6.67 | 0.00 | δ5.0-9.0 20H |
| | | | | | | | δ0.5-4.5 28H |
| 18 | 82.65 | 6.55 | 0.00 | 82.67 | 6.53 | 0.00 | 65.0-9.0 20H |
| | | | | | | | δ0.5-4.5 28H |
| 19 | 81.44 | 6.92 | 0.00 | 81.47 | 6.98 | 0.00 | δ5.0-9.0 15H |
| | | | | | | | δ0.5-4.5 33H |
| 20 | 81.51 | 7.08 | 0.00 | 81.55 | 7.13 | 0.00 | δ5.0-9.0 15H |
| | | | | | | | δ0.5-4.5 35H |
| 21 | 81.67 | 7.22 | 0.00 | 81.63 | 7.27 | 0.00 | δ5.0-9.0 15H |
| | | | | | | | δ0.5-4.5 37H |
| 22 | 81.68 | 7.18 | 2.03 | 81.67 | 7.28 | 1.98 | δ5.0-9.0 15H |
| | | | | | | | δ0.5-4.5 36H |
| 23 | 75.66 | 6.19 | 1.83 | 75.68 | 6.22 | 1.88 | δ5.0-9.0 15H |
| | | | | | | | δ0.5-4.5 31H |
| 24 | 79.40 | 6.74 | 1.98 | 79.40 | 6.66 | 2.01 | δ5.0-9.0 15H |
| | | | | | | | δ0.5-4.5 31H |
| 25 | 81.99 | 7.20 | 0.00 | 81.93 | 7.15 | 0.00 | δ5.0-9.0 15H |
| | | | | | | | δ0.5-4.5 37H |
| 26 | 82.63 | 6.33 | 0.00 | 82.62 | 6.38 | 0.00 | δ5.0-9.0 19H |
| | | | | | | | δ0.5-4.5 27H |
| 27 | 79.88 | 6.55 | 0.00 | 79.86 | 6.56 | 0.00 | δ5.0-9.0 15H |
| | | | | | | | δ0.5-4.5 31H |
| 28 | 77.30 | 6.28 | 0.00 | 77.29 | 6.20 | 0.00 | δ5.0-9.0 15H |
| | | | | | | | δ0.5-4.5 29H |
| 29 | 81.50 | 7.11 | 0.00 | 81.55 | 7.13 | 0.00 | δ5.0-9.0 15H |
| | | | | | | | δ0.5-4.5 35H |
| 30 | 81.54 | 7.29 | 0.00 | 81.63 | 7.27 | 0.00 | δ5.0-9.0 15H |
| | | | | | | | δ0.5-4.5 37H |
| 31 | 80.89 | 6.34 | 0.00 | 80.84 | 6.24 | 0.00 | δ5.0-9.0 19H |
| | | | | | | | δ0.5-4.5 27H |
| 32 | 77.20 | 6.30 | 0.00 | 77.29 | 6.20 | 0.00 | δ5.0-9.0 15H |
| | | | | | | | δ0.5-4.5 29H |
| 33 | 77.38 | 6.28 | 0.00 | 77.29 | 6.20 | 0.00 | δ5.0-9.0 15H |
| | | | | | | | δ0.5-4.5 29H |
| 34 | 70.70 | 5.49 | 0.00 | 70.75 | 5.44 | 0.00 | δ5.0-9.0 15H |
| | | | | | | | δ0.5-4.5 29H |
| 35 | 79.45 | 6.55 | 0.00 | 79.39 | 6.51 | 0.00 | δ5.0-9.0 17H |
| | | | | | | | δ0.5-4.5 27H |
| 36 | 79.55 | 6.76 | 0.00 | 79.51 | 6.67 | 0.00 | δ5.0-9.0 15H |
| | | | | | | | δ0.5-4.5 31H |
| 37 | 81.99 | 7.00 | 2.01 | 81.90 | 7.02 | 1.99 | δ5.0-9.0 16H |
| | | | | | | | δ0.5-4.5 33H |
| 38 | 80.29 | 6.93 | 1.87 | 80.19 | 7.00 | 1.91 | δ5.0-9.0 15H |
| | | | | | | | δ0.5-4.5 36H |
| 39 | 75.67 | 6.00 | 0.00 | 75.58 | 6.07 | 0.00 | δ5.0-9.0 14H |
| | | | | | | | δ0.5-4.5 31H |
| 40 | 80.12 | 6.43 | 2.01 | 80.20 | 6.44 | 1.99 | δ5.0-9.0 14H |
| | | | | | | | δ0.5-4.5 31H |
| 41 | 81.22 | 6.44 | 0.00 | 81.20 | 6.50 | 0.00 | δ5.0-9.0 15H |
| | | | | | | | δ0.5-4.5 27H |
| 42 | 81.41 | 6.63 | 0.00 | 81.30 | 6.67 | 0.00 | δ5.0-9.0 15H |
| | | | | | | | δ0.5-4.5 29H |
| 43 | 81.33 | 6.71 | 0.00 | 81.30 | 6.67 | 0.00 | δ5.0-9.0 15H |
| | | | | | | | δ0.5-4.5 29H |
| 44 | 81.33 | 6.77 | 0.00 | 81.30 | 6.67 | 0.00 | δ5.0-9.0 15H |
| | | | | | | | δ0.5-4.5 29H |
| 45 | 81.11 | 6.11 | 0.00 | 81.00 | 6.15 | 0.00 | δ5.0-9.0 15H |
| | | | | | | | δ0.5-4.5 23H |
| 46 | 78.00 | 5.44 | 0.00 | 78.06 | 5.52 | 0.00 | δ5.0-9.0 15H |
| | | | | | | | δ0.5-4.5 17H |
| 47 | 78.21 | 5.67 | 0.00 | 78.24 | 5.72 | 0.00 | δ5.0-9.0 15H |
| | | | | | | | δ0.5-4.5 19H |
| 48 | 78.41 | 6.39 | 1.87 | 78.42 | 6.30 | 1.94 | δ5.0-9.0 14H |
| | | | | | | | δ0.5-4.5 31H |
| 49 | 78.20 | 5.67 | 0.00 | 78.24 | 5.72 | 0.00 | δ5.0-9.0 15H |
| | | | | | | | δ0.5-4.5 19H |
| 50 | 81.90 | 5.36 | 0.00 | 81.88 | 5.43 | 0.00 | δ5.0-9.0 19H |
| | | | | | | | δ0.5-4.5 15H |
| 51 | 78.10 | 5.61 | 0.00 | 78.06 | 5.52 | 0.00 | δ5.0-9.0 17H |
| | | | | | | | δ0.5-4.5 15H |

### Examples 52 to 92

Photochromic plastic lenses were manufactured and their characteristic properties were evaluated in the same manner as in Example 6 except that the compounds obtained in Examples 11 to 51 were used as chromene compounds. The results are shown in Table 7. In Table 7, compound Nos. 11 to 51 are chromene compounds obtained in Examples 11 to 51, respectively.

**Table 7**

| Example No. | Compound No. | λmax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y} /A_{B} | τ1/2 (sec) | (nm) | (%) | (A₅₀/A₀)X 100 |
| 52 | 11 | 465 | 0.67 | 1.81 | 96 | 413 | 85 | 84 |
| | | 570 | 0.37 | | 96 | | 86 | 84 |
| 53 | 12 | 469 | 0.80 | 1.95 | 102 | 415 | 83 | 83 |
| | | 575 | 0.41 | | 102 | | 84 | 84 |
| 54 | 13 | 463 | 0.60 | 1.71 | 82 | 410 | 86 | 87 |
| | | 567 | 0.35 | | 82 | | 87 | 87 |
| 55 | 14 | 471 | 0.85 | 2.02 | 108 | 417 | 83 | 82 |
| | | 578 | 0.42 | | 109 | | 84 | 82 |
| 56 | 15 | 473 | 0.84 | 2.00 | 107 | 416 | 84 | 80 |
| | | 580 | 0.42 | | 107 | | 85 | 80 |
| 57 | 16 | 442 | 0.40 | 1.33 | 56 | 400 | 88 | 88 |
| | | 559 | 0.30 | | 56 | | 89 | 88 |
| 58 | 17 | 440 | 0.39 | 1.30 | 53 | 410 | 87 | 87 |
| | | 556 | 0.30 | | 54 | | 88 | 87 |
| 59 | 18 | 463 | 0.63 | 1.75 | 86 | 411 | 86 | 86 |
| | | 568 | 0.36 | | 86 | | 86 | 86 |
| 60 | 19 | 446 | 0.63 | 1.91 | 100 | 401 | 86 | 87 |
| | | 558 | 0.33 | | 100 | | 87 | 88 |
| 61 | 20 | 446 | 0.52 | 1.58 | 68 | 403 | 87 | 88 |
| | | 567 | 0.33 | | 69 | | 88 | 88 |
| 62 | 21 | 446 | 0.65 | 1.97 | 101 | 401 | 85 | 88 |
| | | 557 | 0.33 | | 101 | | 87 | 88 |
| 63 | 22 | 478 | 0.78 | 2.36 | 108 | 415 | 81 | 82 |
| | | 553 | 0.33 | | 108 | | 82 | 82 |
| 64 | 23 | 459 | 0.58 | 1.71 | 85 | 407 | 83 | 81 |
| | | 564 | 0.34 | | 86 | | 84 | 81 |
| 65 | 24 | 460 | 0.60 | 1.76 | 90 | 408 | 83 | 81 |
| | | 563 | 0.34 | | 91 | | 84 | 81 |
| 66 | 25 | 446 | 0.51 | 1.55 | 69 | 403 | 87 | 88 |
| | | 567 | 0.33 | | 69 | | 88 | 88 |
| 67 | 26 | 465 | 0.47 | 1.38 | 65 | 416 | 84 | 84 |
| | | 595 | 0.34 | | 65 | | 85 | 84 |
| 68 | 27 | 447 | 0.62 | 2.00 | 103 | 402 | 84 | 85 |
| | | 558 | 0.31 | | 103 | | 85 | 85 |
| 69 | 28 | 446 | 0.55 | 1.62 | 71 | 403 | 86 | 86 |
| | | 567 | 0.34 | | 71 | | 87 | 86 |
| 70 | 29 | 446 | 0.50 | 1.47 | 72 | 403 | 85 | 88 |
| | | 567 | 0.34 | | 72 | | 86 | 88 |
| 71 | 30 | 446 | 0.63 | 1.85 | 105 | 401 | 84 | 87 |
| | | 558 | 0.34 | | 105 | | 85 | 87 |
| 72 | 31 | 453 | 0.65 | 1.44 | 109 | 408 | 82 | 83 |
| | | 584 | 0.45 | | 109 | | 83 | 83 |
| 73 | 32 | 446 | 0.48 | 1.45 | 65 | 404 | 87 | 87 |
| | | 567 | 0.33 | | 65 | | 88 | 87 |
| 74 | 33 | 448 | 0.33 | 1.55 | 69 | 403 | 87 | 87 |
| | | 568 | 0.33 | | 69 | | 88 | 87 |
| 75 | 34 | 448 | 0.33 | 1.55 | 70 | 403 | 87 | 86 |
| | | 568 | 0.33 | | 70 | | 88 | 86 |
| 76 | 35 | 458 | 0.63 | 1.34 | 102 | 405 | 86 | 86 |
| | | 568 | 0.47 | | 102 | | 87 | 86 |
| 77 | 36 | 450 | 0.70 | 1.49 | 105 | 403 | 85 | 86 |
| | | 558 | 0.47 | | 105 | | 86 | 86 |
| 78 | 37 | 472 | 0.48 | 1.00 | 73 | 404 | 85 | 86 |
| | | 586 | 0.48 | | 73 | | 85 | 85 |
| 79 | 38 | 475 | 0.42 | 0.98 | 60 | 408 | 82 | 85 |
| | | 589 | 0.43 | | 60 | | 83 | 85 |
| 80 | 39 | 448 | 0.43 | 1.54 | 42 | 403 | 87 | 83 |
| | | 569 | 0.28 | | 42 | | 88 | 83 |
| 81 | 40 | 449 | 0.35 | 1.52 | 33 | 418 | 87 | 83 |
| | | 571 | 0.23 | | 33 | | 88 | 83 |
| 82 | 41 | 446 | 0.71 | 1.54 | 140 | 403 | 84 | 86 |
| | | 566 | 0.46 | | 141 | | 85 | 86 |
| 83 | 42 | 446 | 0.63 | 1.54 | 126 | 403 | 84 | 86 |
| | | 566 | 0.41 | | 126 | | 85 | 86 |
| 84 | 43 | 445 | 0.45 | 1.55 | 62 | 403 | 87 | 84 |
| | | 566 | 0.29 | | 63 | | 88 | 84 |
| 85 | 44 | 447 | 0.48 | 1.50 | 64 | 403 | 87 | 84 |
| | | 567 | 0.32 | | 65 | | 88 | 84 |
| 86 | 45 | 446 | 0.71 | 1.54 | 151 | 403 | 84 | 86 |
| | | 566 | 0.47 | | 151 | | 84 | 86 |
| 87 | 46 | 446 | 0.51 | 1.55 | 73 | 403 | 77 | 77 |
| | | 567 | 0.33 | | 73 | | 78 | 78 |
| 88 | 47 | 446 | 0.51 | 1.55 | 75 | 403 | 77 | 80 |
| | | 567 | 0.33 | | 75 | | 78 | 80 |
| 89 | 48 | 453 | 0.53 | 1.77 | 66 | 405 | 85 | 85 |
| | | 568 | 0.30 | | 66 | | 86 | 85 |
| 90 | 49 | 446 | 0.51 | 1.55 | 75 | 403 | 81 | 80 |
| | | 568 | 0.33 | | 75 | | 82 | 80 |
| 91 | 50 | 487 | 0.64 | 1.39 | 146 | 416 | 68 | 84 |
| | | 597 | 0.46 | | 146 | | 69 | 84 |
| 92 | 51 | 458 | 0.84 | 1.35 | 176 | 405 | 67 | 86 |
| | | 568 | 0.62 | | 176 | | 68 | 86 |

### Examples of the naphthol compounds are given below.

### Example 93

32 g (193.8 mmol) of a benzene derivative represented by the following formula (22) and 16 g of Wakogel C-300 (of Wako Pure Chemical Industries, Ltd.) were dissolved in 2,300 ml of dichloromethane, the resulting solution was cooled to -15°C, and 34 g (189.9 mmol) of N-bromosuccinimide was added and stirred for 12 hours. After the reaction, the reaction product was washed in water, the solvent was removed, and the obtained product was purified by column chromatography to obtain a bromo material represented by the following formula (23) as 44.8 g (184.1 mmol, yield rate of 95 %) of orange oil.

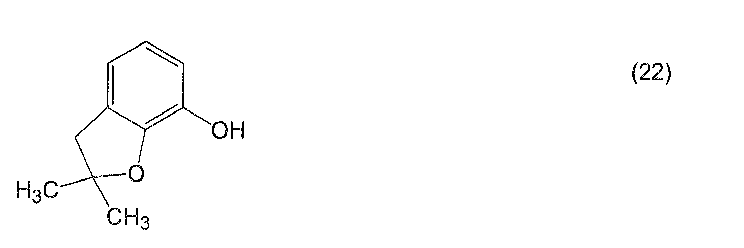

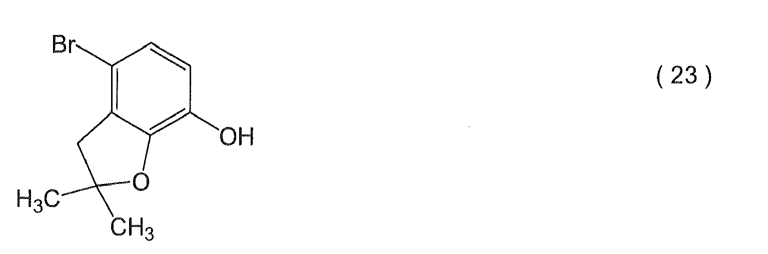

The bromo material of the above formula (23) was dissolved in 900 ml of N,N-dimethylformamide and cooled with ice, and8.1g (202.5 mmol) of sodium hydroxide (60 % in oil) was added and stirred for 1 hour. After agitation, 28.7 g (202.5 mmol) of methyl iodide was added and stirred at room temperature for 5 hours. After the reaction, the reaction product was washed in water, the solvent was removed, and the obtained product was purified by column chromatography to obtain an anisole material represented by the following formula (24) as 45.4 g (176.7 mmol, yield of 96 %) of orange oil.

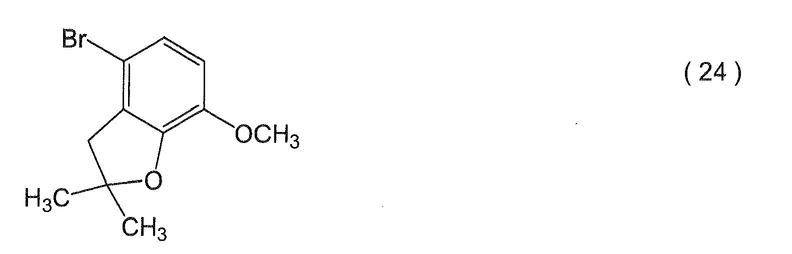

5.2 g (214.0 mmol) of magnesium was added to 230 ml of tetrahydrofuran and heated to 55°C. A tetrahydrofuran (230 ml) solution of the anisole material of the above formula (24) was added dropwise to the above solution to prepare a Grignard reagent. The obtained Grignard reagent was cooled to -78°C, and a tetrahydrofuran (230 ml) solution of 29.8 g (214.0 mmol) of benzoyl chloride was added dropwise to the reagent. After addition, the resulting solution was heated to room temperature and stirred for 3 hours. After the reaction, the reaction product was washed in water, the solvent was removed, and the obtained product was purified by recrystallization with methanol to obtain a benzophenone derivative represented by the following formula (25) as 39.9 g (141.4 mmol; yield rate of 80 %) of a yellow solid.

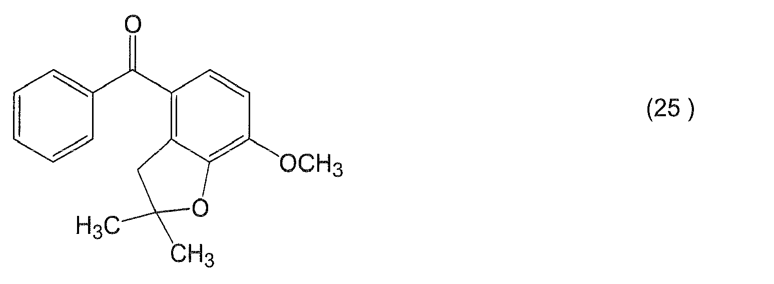

The benzophenone derivative of the above formula (25), and 28.3 g (162.6 mmol) of diethyl succinate were dissolved in 250 ml of tetrahydrofuran and heated to 55°C. A tetrahydrofuran solution (250 ml) of 18.2 g (162.6 mmol) of potassium-t-butoxide was added dropwise to this solution and stirred for 1 hour. After the reaction, the reaction product was washed with concentrated hydrochloric acid and then with water, the solvent was removed, and the obtained product was purified by column chromatography to obtain a stobbe material represented by the following formula (26) as 58.0 g (141.4 mmol; yield rate of 100 %) of orange oil.

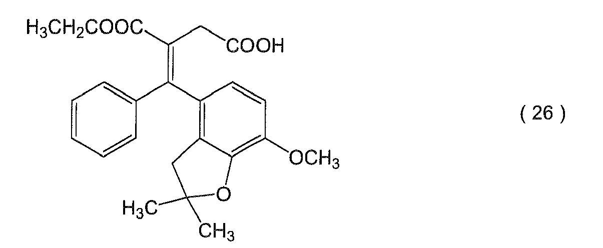

The above stobbe material of the above formula (26), 11. 6 g (141.4 mmol) of sodium acetate and 72.2 g (707.0 mmol) of acetic anhydride were dissolved in 180 ml of toluene and refluxed for 3 hours. After the reaction, the reaction product was washed in water, the solvent was removed, and the reaction product was purified by recrystallization with methanol to obtain an acetyl material represented by the following formula (27) as 21.5 g (49.5 mmol, yield rate of 35 %) of an orange solid.

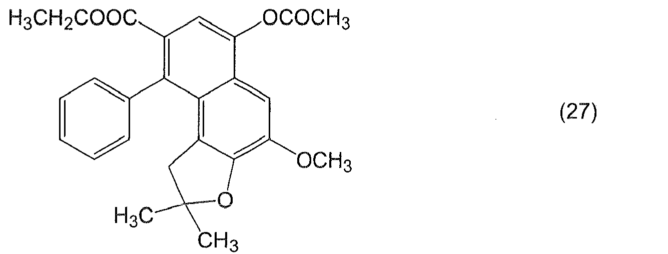

The acetyl material of the above formula (27) was dispersed into 80 ml of methanol. 300 ml of an aqueous solution of 35.6 g (891.0 mmol) of sodium hydroxide was added to this solution and refluxed for 3 hours. After the reaction, the reaction product was washed with concentrated hydrochloric acid and then with water, the solvent was removed, and the obtained product was purified by reslurrying with toluene to obtain a hydroxycarboxylic acid derivative represented by the following formula (28) as 16.2 g (44.6 mmol, yield rate of 90 %) of a yellow solid.

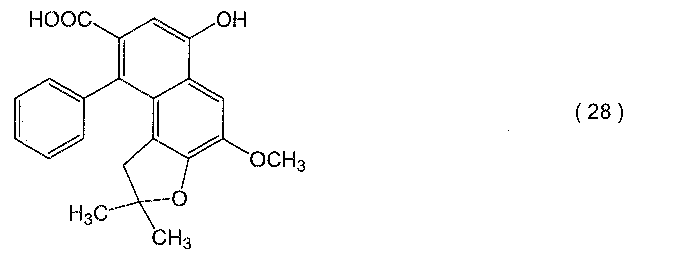

The hydroxycarboxylic acid derivative of the above formula (28) and 12.4 g (98.1 mmol) of benzyl chloride were dissolved in 160 ml of N,N-dimethylformamide. 15.4 g (111.5 mmol) of potassium carbonate was added to this solution, heated to 60 °C and stirred for 3 hours. After the reaction, the reaction product was washed in water, and the solvent was removed to obtain a dibenzyl material represented by the following formula (29) as 23.8 g (43.7 mmol, yield rate of 98 %) of yellow oil.

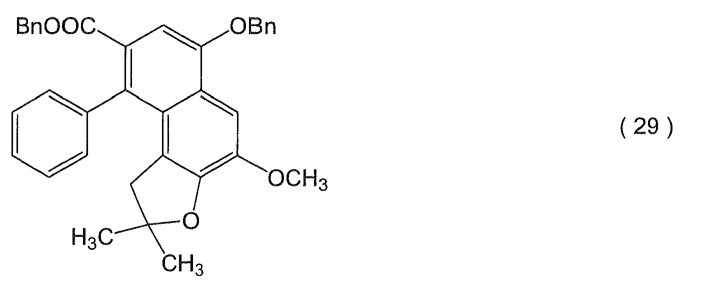

The dibenzyl material of the above formula (29) was dispersed into 150 ml of isopropyl alcohol. 120 ml of an aqueous solution of 26.2 g (655.5 mmol) of sodium hydroxide was added to this solution and refluxed for 3 hours. After the reaction, the reaction product was washed with concentrated hydrochloric acid and then with water, the solvent was removed, and the obtained product was purified by reslurrying with toluene to obtain a benzyloxycarboxylic acid derivative represented by the following formula (30) as 17.3 g (38.0 mmol, yield rate of 87 %) of a yellow solid.

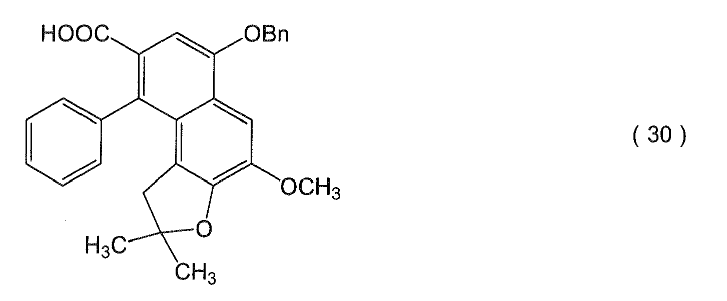

The benzyloxycarboxylic acid derivative of the above formula (30) was dispersed into 350 ml of toluene. 11.5 (114.0 mmol) of triethylamine and 13.6 g (49.4 mmol) of diphenylphosphorylazide were added to this solution and stirred at room temperature for 2 hours. 8.8 g (190.0 mmol) of ethanol was added to this solution to carry out a reaction at 70°C for 2 hours. Thereafter, 100 ml of ethanol was added to this solution, and then 21.3 g (380.0 mmol) of potassium hydroxide was added and refluxed for 5 hours. After the reaction, ethanol was distilled off at normal pressure, tetrahydrofuran was added, the solution was washed in water, and the solvent was removed to obtain an amino material represented by the following formula (31) as 16.2 g (38.0 mmol, yield rate of 100 %) of a yellow solid.

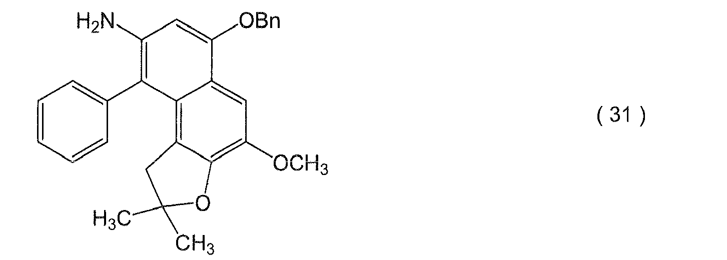

The amino material of the above formula (31) was dispersed into 500 ml of acetonitrile, and 120g (190.0 mmol) of a 6 % hydrochloric acid aqueous solution was added to the dispersion and cooled to 0 to 5°C. 23.6 g (114.0 mmol) of a 33 % sodium nitrite aqueous solution was added to this solution and stirred for 30 minutes. 66.1 g (190.0 mmol) of a 50 % potassium iodide aqueous solution was added to this solution and stirred at room temperature for 5 hours. After the reaction, toluene was added, the reaction product was washed in water, the solvent was removed, and the obtained product was purified by column chromatography to obtain an iodine material represented by the following formula (32) as 20.4 g (27.4 mmol, yield rate of 72 %) of a yellow solid.

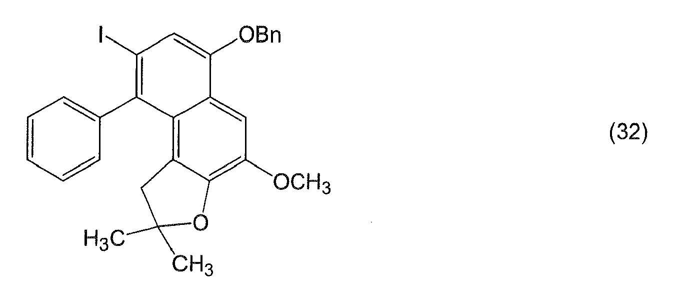

The iodine material of the above formula (32) was dispersed into 600 ml of toluene and cooled to -30°C. 20.6 ml (32.9 mmol) of n-butyl lithium (1. 6M hexane solution) was added dropwise to this solution and stirred for 30 minutes. 10.6 ml of a toluene solution of 5.3 g (34.3 mmol) of 3,3,5,5-tetramethylcyclohexanone was added dropwise to this solution and stirred at 0 °C for 3 hours. After the reaction, toluene was added, the reaction product was washed in water, the solvent was removed, and the obtained product was purified by reslurrying with methanol to obtain a ketone adduct represented by the following formula (33) as 10.1 g (17.8 mmol, yield rate of 65 %) of a yellow solid.

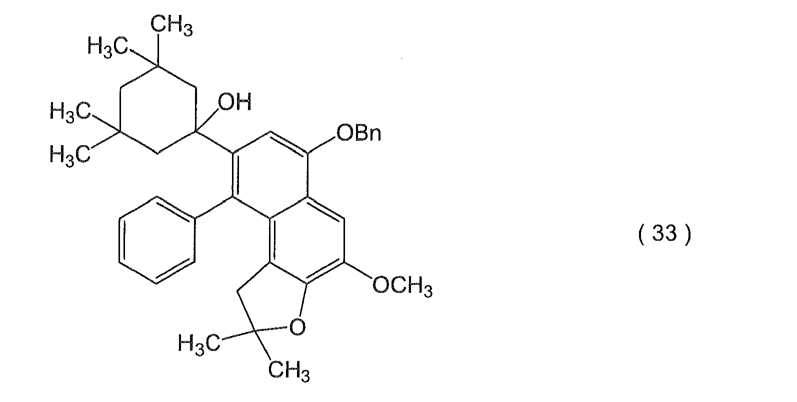

The ketone adduct of the above formula (33) was dissolved in 200 ml of tetrahydrofuran, and 4.5 (71.2 mmol) of ammonium formate and 5 g of 5 % palladium carbide were added and stirred at room temperature for 8 hours. After the reaction, toluene was added, the reaction product was washed in water, the solvent was removed, and the obtained product was purified by reslurrying with toluene to obtain a debenzylated material represented by the following formula (34) as 7.6 g (16.0 mmol, yield rate of 90 %) of a yellow solid.

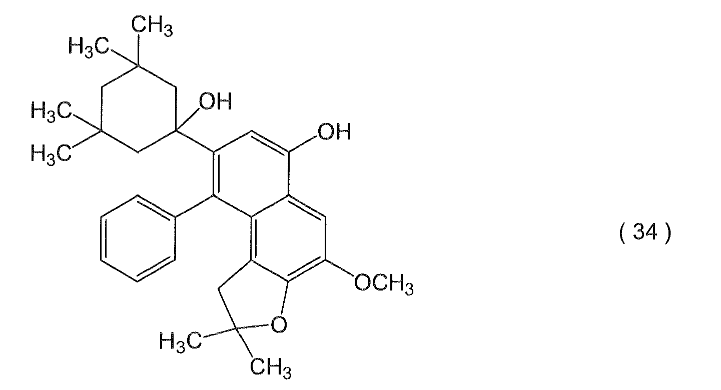

The debenzylated material of the above formula (34) was dissolved in 150 ml of toluene and heated to 90°C. 9.1 g (48.0 mmol) of p-toluensulfonic acid was added to this solution and refluxed for 3 hours. After the reaction, the reaction product was washed in water, and the solvent was removed to obtain a naphthol material represented by the following formula (35) as 5.7 g (12.5 mmol, yield rate of 78 %) of a yellow solid.

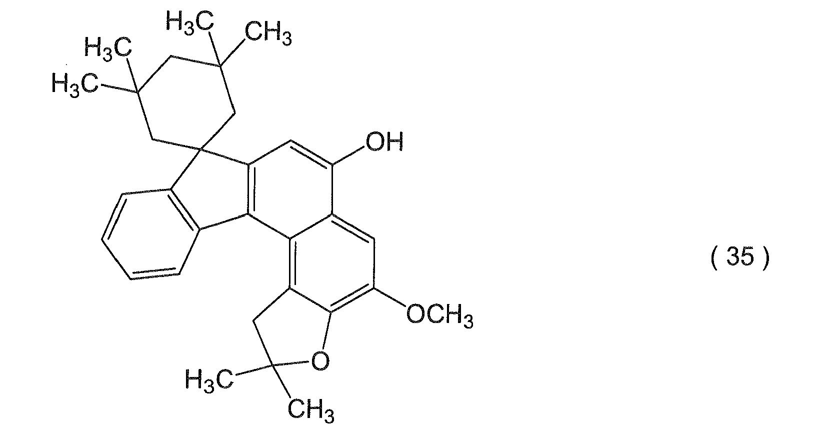

The elemental analysis values of this product were 81.41 % of C, 7.99 % of H and 0.00 % of N which were almost equal to the calculated values of C₃₁H₃₆O₃ (C: 81.54 %, H: 7.95 %, N: 0.00 %).

When the proton nuclear magnetic resonance spectrum was measured, it showed 29H peaks based on an alkyl group and an alkoxy group at δ of around 0.5 to 4.5 ppm, and 7H peaks based on a hydroxyl group and an aromatic proton at δ of around 5.0 to 9.0 ppm

Further, when the ¹³C-nuclear magnetic resonance spectrum was measured, it showed a peak based on the carbon of an aromatic ring at δ of around 110 to 160 ppm, a peak based on the carbon of an alkyleneoxy group at δ of around 80 to 140 ppm, and peaks based on the carbons of an alkyl group and an alkoxy group at δ of around 20 to 80 ppm.

It was confirmed from these results that the isolated product was a compound represented by the above formula (35) .

This compound is a naphthol compound used in the above Example 20.

### Examples 94 to 135

Naphthol compounds shown in the table were synthesized in the same manner as in Example 93. When the structures of the obtained products were analyzed by using the same structure confirming means as in Example 93, it was confirmed that they were naphthol compounds used in Examples shown in Table 8. Table 8 shows the elemental analysis values of these compounds and the calculated values and characteristic ¹H-NMR spectra obtained from the structural formulas of the compounds.

**Table 8**

| Example No. | Examples of chromene compounds* No. | Elemental analysis values | | | | | | ¹H-NMR (NMR) |
|---|---|---|---|---|---|---|---|---|
| | | Experimental values | | | Calculated values | | | |
| | | C | H | N | C | H | N | |
| 94 | 1 | 81.34 | 7.46 | 0.00 | 81.27 | 7.53 | 0.00 | δ5.0-9.0 7H δ0.5-4.5 25H |
| 95 | 2 | 79.43 | 6.00 | 0.00 | 79.50 | 6.06 | 0.00 | δ5.0-9.0 7H δ0.5-4.5 13H |
| 96 | 3 | 81.52 | 8.01 | 3.25 | 81.59 | 7.99 | 3.17 | δ5.0-9.0 7H δ0.5-4.5 28H |
| 97 | 5 | 71.76 | 5.77 | 0.00 | 71.78 | 5.81 | 0.00 | δ5.0-9.0 6H δ0.5-4.5 21H |
| 98 | 11 | 79.42 | 7.77 | 2.86 | 79.47 | 7.71 | 2.90 | δ5.0-9.0 7H δ0.5-4.5 30H |
| 99 | 12 | 82.34 | 8.09 | 2.91 | 82.29 | 8.16 | 2.91 | δ5.0-9.0 7H δ0.5-4.5 32H |
| 100 | 13 | 81.11 | 7.34 | 0.00 | 81.13 | 7.29 | 0.00 | δ5.0-9.0 8H δ0.5-4.5 22H |
| 101 | 14 | 81.67 | 8.00 | 3.14 | 81.59 | 7.99 | 3.17 | δ5.0-9.0 7H δ0.5-4.5 28H |
| 102 | 15 | 81.29 | 7.61 | 3.33 | 81.32 | 7.56 | 3.38 | δ5.0-9.0 7H δ0.5-4.5 24H |
| 103 | 16 | 84.46 | 7.88 | 0.00 | 84.43 | 7.82 | 0.00 | δ5.0-9.0 7H δ0.5-4.5 25H |
| 104 | 17 | 86.11 | 7.25 | 0.00 | 86.04 | 7.22 | 0.00 | δ5.0-9.0 12H δ0.5-4.5 22H |
| 105 | 18 | 83.33 | 7.02 | 0.00 | 83.23 | 6.98 | 0.00 | δ5.0-9.0 12H δ0.5-4.5 22H |
| 106 | 19 | 81.44 | 7.67 | 0.00 | 81.41 | 7.74 | 0.00 | δ5.0-9.0 7H δ0.5-4.5 27H |
| 93 | 20 | 81.61 | 7.99 | 0.00 | 81.54 | 7.95 | 0.00 | δ5.0-9.0 7H δ0.5-4.5 29H |
| 107 | 21 | 81.74 | 8.15 | 0.00 | 81.66 | 8.14 | 0.00 | δ5.0-9.0 7H δ0.5-4.5 31H |
| 108 | 22 | 81.77 | 8.20 | 2.97 | 81.72 | 8.19 | 3.07 | δ5.0-9.0 7H δ0.5-4.5 30H |
| 109 | 23 | 72.77 | 6.55 | 2.81 | 72.71 | 6.51 | 2.83 | δ5.0-9.0 7H δ0.5-4.5 19H |
| 110 | 24 | 78.11 | 7.26 | 3.11 | 78.17 | 7.24 | 3.14 | δ5.0-9.0 7H δ0.5-4.5 25H |
| 111 | 25 | 82.22 | 7.89 | 0.00 | 82.12 | 7.94 | 0.00 | δ5.0-9.0 7H δ0.5-4.5 31H |
| 112 | 26 | 83.11 | 6.79 | 0.00 | 83.16 | 6.77 | 0.00 | δ5.0-9.0 11H δ0.5-4.5 21H |
| 113 | 27 | 78.99 | 6.99 | 0.00 | 78.92 | 7.06 | 0.00 | δ5.0-9.0 7H δ0.5-4.5 25H |
| 114 | 28 | 74.94 | 6.48 | 0.00 | 74.98 | 6.51 | 0.00 | δ5.0-9.0 7H δ0.5-4.5 23H |
| 115 | 29 | 81.65 | 7.89 | 0.00 | 81.54 | 7.95 | 0.00 | δ5.0-9.0 7H δ0.5-4.5 29H |
| 116 | 30 | 81.61 | 8.21 | 0.00 | 81.66 | 8.14 | 0.00 | δ0.5-9.0 7H δ0.5-4.5 31H |
| 117 | 31 | 80.55 | 6.44 | 0.00 | 80.46 | 6.55 | 0.00 | δ5.0-9.0 11H δ0.5-4.5 15H |
| 118 | 32 | 74.99 | 6.55 | 0.00 | 74.98 | 6.51 | 0.00 | δ5.0-9.0 7H δ0.5-4.5 23H |
| 119 | 33 | 74.89 | 6.51 | 0.00 | 74.98 | 6.51 | 0.00 | δ5.0-9.0 7H δ0.5-4.5 23H |
| 120 | 34 | 65.91 | 5.43 | 0.00 | 65.95 | 5.36 | 0.00 | δ5.0-9.0 7H δ0.5-4.5 23H |
| 121 | 35 | 78.08 | 7.00 | 0.00 | 78.11 | 7.02 | 0.00 | δ5.0-9.0 9H δ0.5-4.5 21H |
| 122 | 36 | 78.28 | 7.22 | 0.00 | 78.35 | 7.26 | 0.00 | δ5.0-9.0 7H δ0.5-4.5 25H |
| 123 | 39 | 72.52 | 6.33 | 0.00 | 72 56 | 6.29 | 0.00 | δ5.0-9.0 6H δ0.5-4.5 25H |
| 124 | 40 | 79.45 | 6.93 | 3.07 | 79.44 | 6.89 | 3.09 | δ5.0-9.0 6H δ0.5-4.5 25H |
| 125 | 41 | 80.99 | 7.12 | 0.00 | 80.97 | 7.05 | 0.00 | δ5.0-9.0 7H δ0.5-4.5 21H |
| 126 | 42 | 81.20 | 7.19 | 0.00 | 81.13 | 7.29 | 0.00 | δ5.0-9.0 7H δ0.5-4.5 23H |
| 127 | 43 | 81.11 | 7.30 | 0.00 | 81.13 | 7.29 | 0.00 | δ5.0-9.0 7H δ0.5-4.5 23H |
| 128 | 44 | 81.07 | 7.33 | 0.00 | 81.13 | 7.29 | 0.00 | δ5.0-9.0 7H δ0.5-4.5 23H |
| 129 | 45 | 80.52 | 6.49 | 0.00 | 80.62 | 6.49 | 0.00 | δ5.0-9.0 7H δ0.5-4.5 17H |
| 130 | 46 | 75.34 | 5.41 | 0.00 | 75.43 | 5.43 | 0.00 | δ5.0-9.0 7H δ0.5-4.5 11H |
| 131 | 47 | 75.88 | 5.87 | 0.00 | 75.84 | 5.79 | 0.00 | δ5.0-9.0 7H δ0.5-4.5 13H |
| 132 | 48 | 81.18 | 6.19 | 0.00 | 81.22 | 6.29 | 0.00 | δ5.0-9.0 7H δ0.5-4.5 17H |
| 133 | 49 | 75.90 | 5.74 | 0.00 | 75.84 | 5.79 | 0.00 | δ5.0-9.0 7H δ0.5-4.5 13H |
| 134 | 50 | 82.00 | 5.33 | 0.00 | 82.08 | 5.30 | 0.00 | δ5.0-9.0 11H δ0.5-4.5 9H |
| 135 | 51 | 75.39 | 5.49 | 0.00 | 75.43 | 5.43 | 0.00 | δ5.0-9.0 9H δ0.5-4.5 9H |

| | | | | | | | | |
|---|---|---|---|---|---|---|---|---|
| *Examples of chromene compounds produced by using the naphthol compounds of Examples. | | | | | | | | |

### Effect of the Invention

Since the chromene compound of the present invention develops a color of a neutral tint by itself, it can be used alone and hardly undergoes a color change at the time of fading and a color change at the time of deterioration. Further, since it has little initial coloration, high color optical density, high double peak characteristic and high fading speed, an extremely excellent photochromic lens can be obtained from the chromene compound. Therefore, color can be controlled by mixing it with another photochromic compound, and even when it is mixed with another photochromic compound, it can exhibit excellent photochromic properties.

## Claims

1. A chromene compound having a skeleton represented by the following formula (1) : 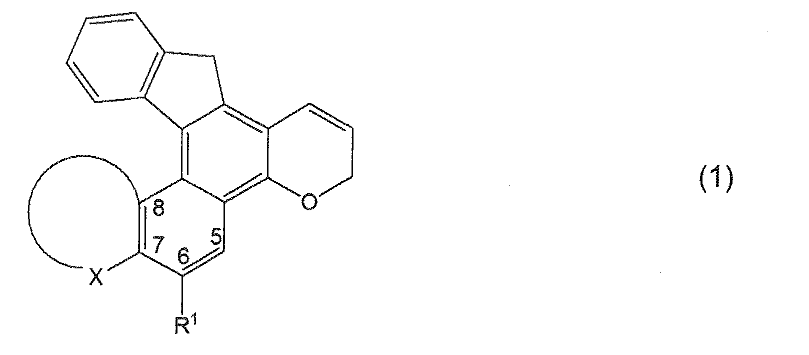 wherein R¹ is an electron donor group having a Hammett constant σₚ of less than 0, and the ring portion represented by the following formula (2) is a hetero ring: 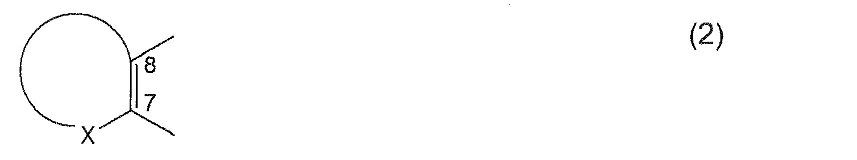 wherein X is an oxygen atom, sulfur atom or group represented by the following formula (3) which is directly bonded to the 7-position carbon atom: 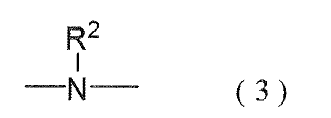 wherein R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom.

2. The chromene compound according to claim 1, wherein the hetero ring represented by the formula (2) is a hetero ring represented by the following formula (4): 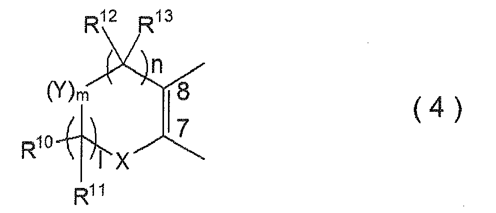 wherein X is as defined in the above formula (2), and Y is an oxygen atom, sulfur atom or group represented by the following formula (3): 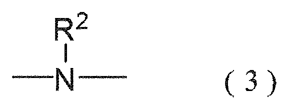 wherein R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom, R¹⁰, R¹¹, R¹² and R¹³ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the carbon atom bonded thereto, cyano group, nitro group, formyl group, hydroxycarbonyl group, alkylcarbonyl group, alkoxycarbonyl group or halogen atom, R¹⁰ and R¹¹, or R¹² and R¹³ may be bonded together to form a carbonyl group or aliphatic hydrocarbon ring together with the carbon atom bonded thereto, 1 is an integer of 0 to 4, m is an integer of 0 to 1, n is an integer of 0 to 4, and (1+m+n) is an integer of 2 to 4, with the proviso that when m is 0, 1 is an integer of 0 to 4, n is an integer of 0 to 4, and (1+n) is an integer of 2 to 4, and in this case, adjacent R¹⁰ and R¹⁰ (1 ≠ 0 and 1), R¹⁰ and R¹² (1 ≠ 0 and n ≠ 0), or R¹² and R¹² (n ≠ 0 and 1) may be bonded together to form an aliphatic hydrocarbon ring or aromatic hydrocarbon ring together with the carbon atom bonded thereto, and when m is 1, 1 is an integer of 0 to 3, n is an integer of 0 to 3, and (1+n) is an integer of 2 to 3, and in this case, adjacent R¹⁰ and R¹⁰ (1 ≠ 0 and 1) or adjacent R¹² and R¹² (n ≠ 0 and 1) may be bonded together to form an aliphatic hydrocarbon ring or aromatic hydrocarbon ring together with the carbon atom bonded thereto;
and the chromene compound has a skeleton represented by the following formula (5): 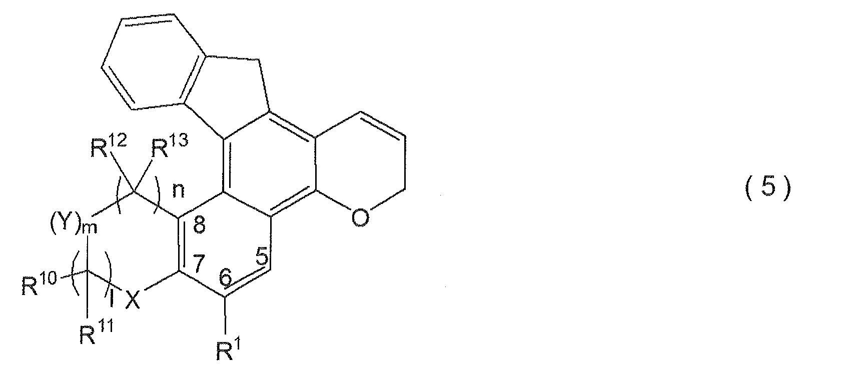 wherein R¹ and X are as defined in the above formula (1) and R¹⁰, R¹¹, R¹², R¹³, Y, 1, m and n are as defined in the above formula (4).

3. The chromene compound according to claim 1 which is represented by the following formula (6): 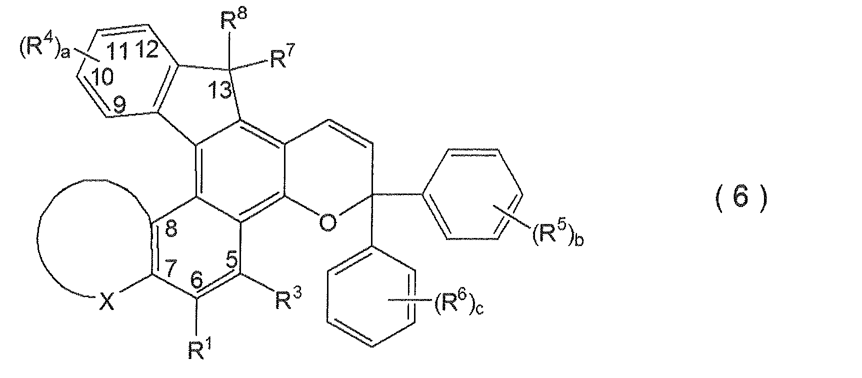 wherein R¹ is, the ring portion represented by the following formula and X are as defined in the above formula (1): 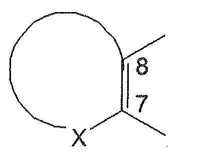 R³ is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 5-position carbon atom, cyano group, nitro group, formyl group, hydroxycarbonyl group, alkylcarbonyl group, alkoxycarbonyl group or halogen atom, R⁴, R⁵ and R⁶ are each independently a hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the benzene ring bonded thereto, cyano group, nitro group, formyl group, hydroxycarbonyl group, alkylcarbonyl group, alkoxycarbonyl group or halogen atom, a is an integer of 0 to 4, b and c are each independently an integer of 0 to 5, with the proviso that when a is 2 or more, R⁴'s may be the same or different and when b and c are each 2 or more, R⁵'s may be the same
or different and R⁶'s may be the same or different, R⁷ and R⁸ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 13-position carbon atom, cyano group, nitro group, formyl group, hydroxycarbonyl group, alkylcarbonyl group, alkoxycarbonyl group or halogen atom, and R⁷ and R⁸ may be bonded together to form a carbonyl group or aliphatic hydrocarbon ring together with the 13-position carbon atom.

4. The chromene compound according to claim 2 which is represented by the following formula (7): 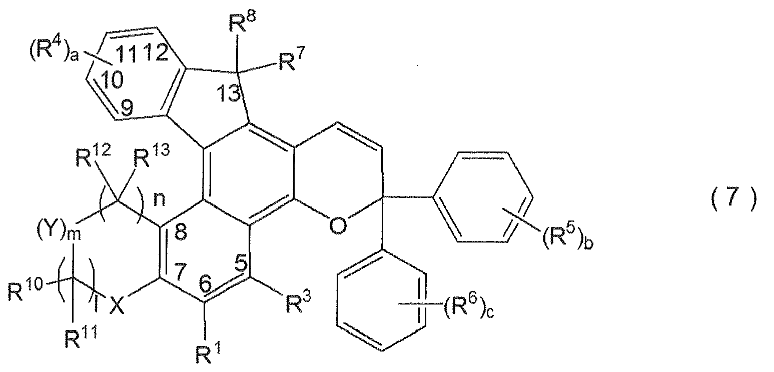 wherein R¹, R³, R⁴, R⁵, R⁶, R⁷, R⁸, a, b and c are as defined in the above formula (6), and the ring portion represented by the following formula and X, Y, R¹⁰, R¹¹, R¹², R¹³, 1, m and n in the above formula are as defined in the above formula (5).

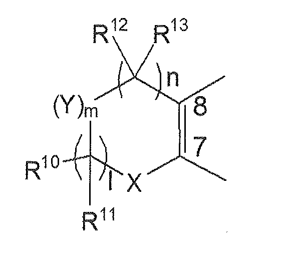

5. The chromene compound according to claim 3 or 4, wherein, in the chromene compound represented by the above formula (6) or (7), R⁷ and R⁸ are bonded together to form an aliphatic hydrocarbon ring together with the 13-position carbon atom, and the aliphatic hydrocarbon ring has 4 to 20 member carbon atoms and may have at least one substituent selected from the group consisting of alkyl group, haloalkyl group, cycloalkyl group, alkoxy group, amino group, aralkyl group, aryl group and halogen atom.

6. A photochromic curable composition comprising the chromene compound of any one of claims 1 to 5 and a polymerizable monomer.

7. A photochromic optical article having a polymer molded product containing the chromene compound of any one of claims 1 to 5 dispersed therein as a constituent member.

8. An optical article having an optical substrate whose surface is at least partially coated with a polymer film as a constituent part, wherein the polymer film contains the chromene compound of any one of claims 1 to 5 dispersed therein.

9. A naphthol compound represented by the following formula (8): 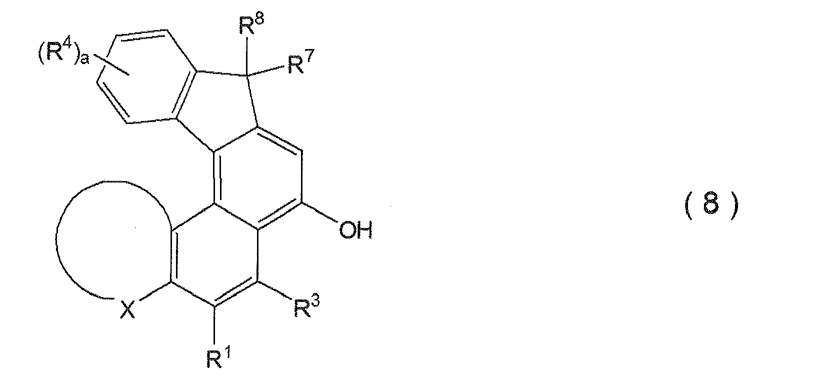 wherein a, R¹, R³, R⁴, R⁷, R⁸ and a ring portion including X are as defined in the above formula (6).

## Amended claims

### Amended claims under Art. 19.1 PCT

**1.** A chromene compound having a skeleton represented by the following formula (1): 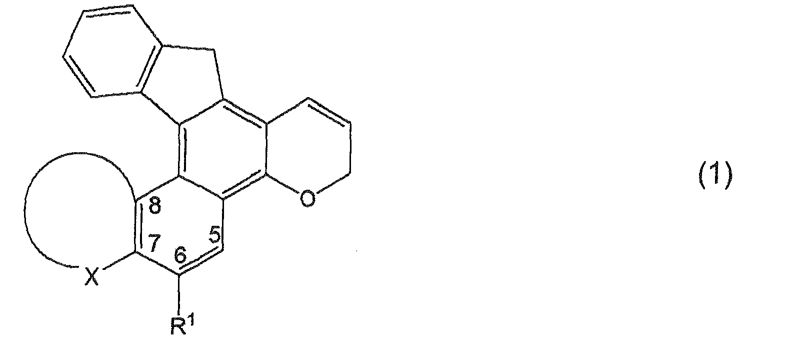 wherein R¹ is an electron donor group having a Hammett constant σₚ of less than 0, and the ring portion represented by the following formula (2) is a hetero ring: 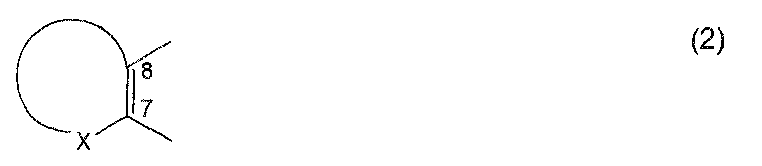 wherein X is an oxygen atom, sulfur atom or group represented by the following formula (3) which is directly bonded to the 7-position carbon atom: 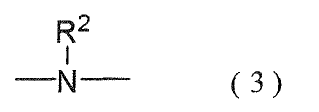 wherein R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom.

**2.** The chromene compound according to claim 1, wherein the hetero ring represented by the formula (2) is a hetero ring represented by the following formula (4): 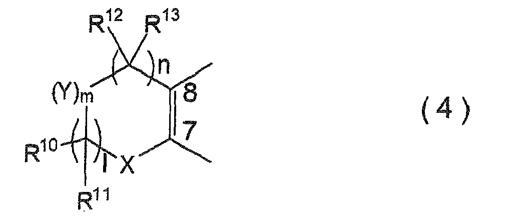 wherein X is as defined in the above formula (2), and Y is an oxygen atom, sulfur atom or group represented by the following formula (3): 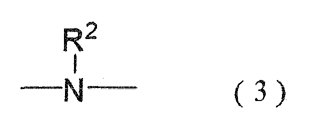 wherein R² is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the nitrogen atom in the above formula (3), cyano group, nitro group, formyl group, hydroxycarbonyl group, alkylcarbonyl group, alkoxycarbonyl group or halogen atom, R¹⁰, R¹¹, R¹² and R¹³ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the carbon atom bonded thereto, cyano group, nitro group, formyl group, hydroxycarbonyl group, alkylcarbonyl group, alkoxycarbonyl group or halogen atom, R¹⁰ and R¹¹, or R¹² and R¹³ may be bonded together to form a carbonyl group or aliphatic hydrocarbon ring together with the carbon atom bonded thereto, 1 is an integer of 0 to 4, m is an integer of 0 to 1, n is an integer of 0 to 4, and (1+m+n) is an integer of 2 to 4, with the proviso that when m is 0, 1 is an integer of 0 to 4, n is an integer of 0 to 4, and (1+n) is an integer of 2 to 4, and in this case, adjacent R¹⁰ and R¹⁰ (1 ≠ 0 and 1), R¹⁰ and R¹² (1 ≠ 0 and n ≠ 0), or R¹² and R¹² (n ≠ 0 and 1) may be bonded together to form an aliphatic hydrocarbon ring or aromatic hydrocarbon ring together with the carbon atom bonded thereto, and when m is 1, 1 is an integer of 0 to 3, n is an integer of 0 to 3, and (1+n) is an integer of 2 to 3, and in this case, adjacent R¹⁰ and R¹⁰ (1 ≠ 0 and 1) or adjacent R¹² and R¹² (n ≠ 0 and 1) may be bonded together to form an aliphatic hydrocarbon ring or aromatic hydrocarbon ring together with the carbon atom bonded thereto;
and the chromene compound has a skeleton represented by the following formula (5): 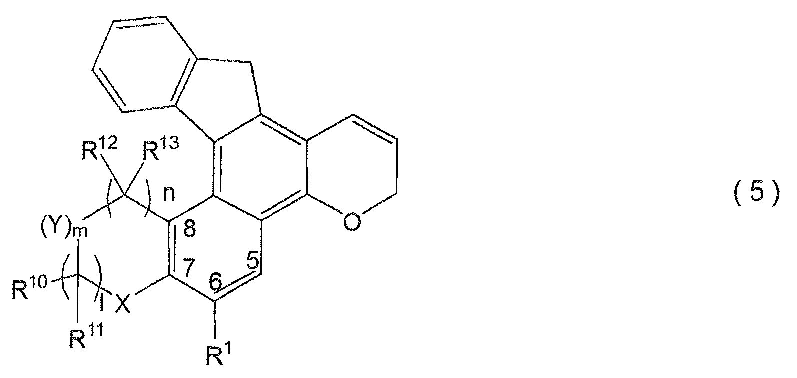 wherein R¹ and X are as defined in the above formula (1) and R¹⁰, R¹¹, R¹², R¹³, Y, 1, m and n are as defined in the above formula (4).

**3.** (amended) The chromene compound according to claim 1 which is represented by the following formula (6): 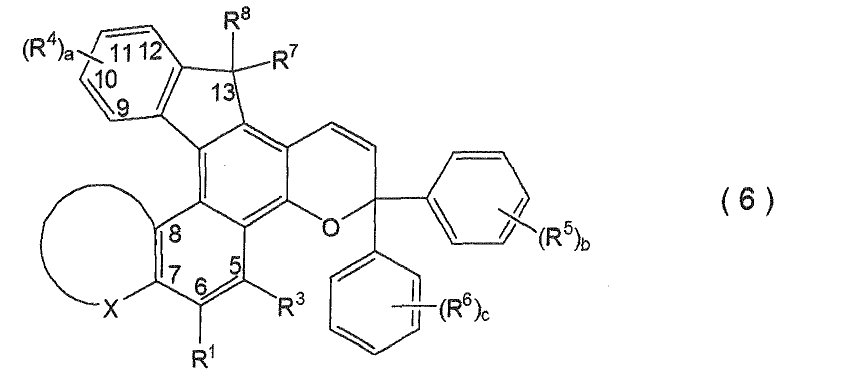 wherein R¹, the ring portion represented by the following formula and X are as defined in the above formula (1): 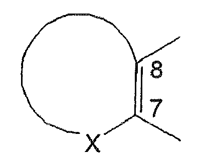 R³ is a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 5-position carbon atom, cyano group, nitro group, formyl group, hydroxycarbonyl group, alkylcarbonyl group, alkoxycarbonyl group or halogen atom, R⁴, R⁵ and R⁶ are each independently a hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the benzene ring bonded thereto, cyano group, nitro group, formyl group, hydroxycarbonyl group, alkylcarbonyl group, alkoxycarbonyl group or halogen atom, a is an integer of 0 to 4, b and c are each independently an integer of 0 to 5, with the proviso that when a is 2 or more, R⁴'s may be the same or different and when b and c are each 2 or more, R⁵'s may be the same or different and R⁶'s may be the same or different, R⁷ and R⁸ are each independently a hydrogen atom, hydroxyl group, alkyl group, haloalkyl group, alkenyl group, alkynyl group, cycloalkyl group, alkoxy group, aralkyl group, aryloxy group, aryl group, amino group, heterocyclic group having a nitrogen atom which nitrogen atom is a ring-membered hetero atom and bonds to the 13-position carbon atom, cyano group, nitro group, formyl group, hydroxycarbonyl group, alkylcarbonyl group, alkoxycarbonyl group or halogen atom, and R⁷ and R⁸ may be bonded together to form a carbonyl group or aliphatic hydrocarbon ring together with the 13-position carbon atom.

**4.** The chromene compound according to claim 2 which is represented by the following formula (7): 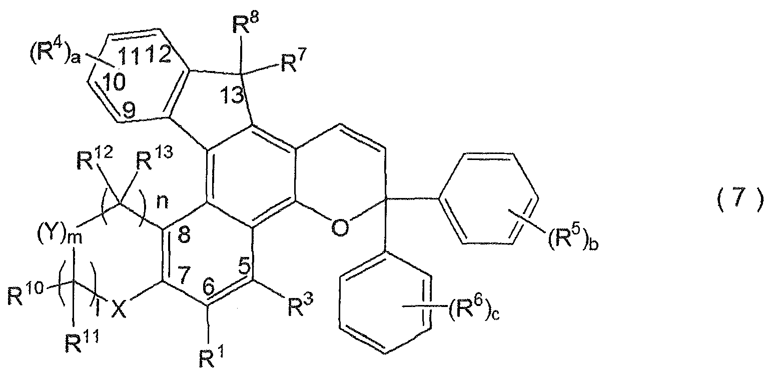 wherein R¹, R³, R⁴, R⁵, R⁶, R⁷, R⁸, a, b and c are as defined in the above formula (6), and the ring portion represented by the following formula and X, Y, R¹⁰, R¹¹, R¹², R¹³, 1, m and n in the above formula are as defined in the above formula (5).

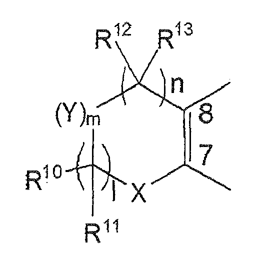

**5.** The chromene compound according to claim 3 or 4, wherein, in the chromene compound represented by the above formula (6) or (7), R⁷ and R⁸ are bonded together to form an aliphatic hydrocarbon ring together with the 13-position carbon atom, and the aliphatic hydrocarbon ring has 4 to 20 member carbon atoms and may have at least one substituent selected from the group consisting of alkyl group, haloalkyl group, cycloalkyl group, alkoxy group, amino group, aralkyl group, aryl group and halogen atom.

**6.** A photochromic curable composition comprising the chromene compound of any one of claims 1 to 5 and a polymerizable monomer.

**7.** A photochromic optical article having a polymer molded product containing the chromene compound of any one of claims 1 to 5 dispersed therein as a constituent member.

**8.** An optical article having an optical substrate whose surface is at least partially coated with a polymer film as a constituent part, wherein the polymer film contains the chromene compound of any one of claims 1 to 5 dispersed therein.

**9.** A naphthol compound represented by the following formula (8): 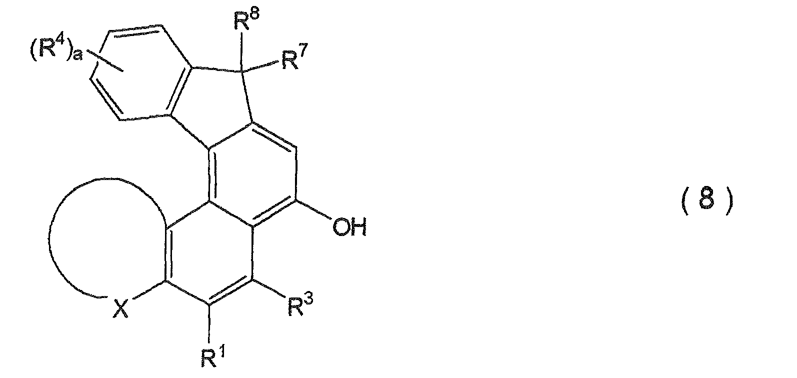 wherein a, R¹, R³, R⁴, R⁷, R⁸ and a ring portion including X are as defined in the above formula (6).
